# EUROPEAN PATENT APPLICATION

(11) **EP 3 181 565 A1**
(43) Date of publication of application: **21.06.2017**
(21) Application number: 15201150.8
(22) Date of filing: 18.12.2015
(51) Int. Cl.: C07D 487/04, A61K 31/4162, A61P 3/10

(54) **CRYSTALLINE OMARIGLIPTIN SALTS**

(71) Applicant: SANDOZ AG, 4056 Basel (CH)
(72) Inventor: LENGAUER, Hannes, A-6250 Kundl (AT); HOTTER, Andreas, A-6250 Kundl (AT)
(74) Representative: Kluschanzoff, Harald

(57) **Abstract**

The present invention relates to non-hygroscopic omarigliptin acid addition salts, preferably to non-hygroscopic crystalline omarigliptin acid addition salts such as omarigliptin acid addition salts with an acid selected from the group of hydrochloric acid, fumaric acid, R-mandelic acid, *S-*mandelic acid and *RS-*mandelic acid and to methods for their preparation. In addition, the invention is directed to pharmaceutical compositions comprising an effective amount of an omarigliptin acid addition salt of the present invention and at least one pharmaceutically acceptable excipient. The pharmaceutical compositions of the present invention are suitable for the treatment of type 2 diabetes, obesity and high blood pressure, in particular for the treatment of type 2 diabetes.

## Description

### FIELD OF THE INVENTION

The present invention relates to non-hygroscopic omarigliptin acid addition salts, preferably to non-hygroscopic crystalline omarigliptin acid addition salts such as omarigliptin acid addition salts with an acid selected from the group of hydrochloric acid, fumaric acid, *R*-mandelic acid, *S-*mandelic acid and *RS*-mandelic acid and to methods for their preparation. In addition, the invention is directed to pharmaceutical compositions comprising an effective amount of an omarigliptin acid addition salt of the present invention and at least one pharmaceutically acceptable excipient. The pharmaceutical compositions of the present invention are suitable for the treatment of type 2 diabetes, obesity and high blood pressure, in particular for the treatment of type 2 diabetes.

### BACKGROUND OF THE INVENTION

Omarigliptin is known under the IUPAC name "(2*R*,3*S*,5*R*)-2-(2,5-difluorophenyl)-5-[2-(methylsulfonyl)-2,6-dihydropyrrolo[3,4-*c*]pyrazol-5(4*H*)-yl]tetrahydro-2*H*-pyran-3 amine" and can be represented by the following chemical structure according to Formula (A):

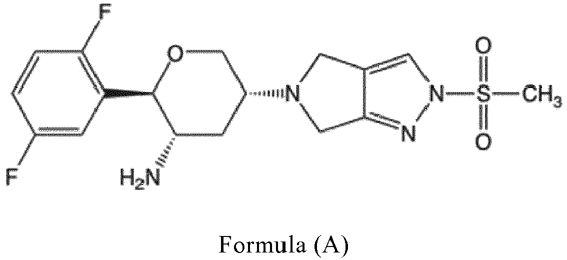

Omarigliptin is reported to belong to the class of dipeptidyl peptidase-IV-inhibitors, also known as DPP-4-inhibitors or "gliptins". While glucagon increases the blood glucose levels, DPP-4 inhibitors are able to reduce glucagon and, thus, also blood glucose levels. The mechanism of DPP-4 inhibitors is to increase the levels of glucagon-like peptide 1 (GLP-1) and glucose-dependent insulinotropic peptide (GIP), both peptides inhibiting glucagon release. In turn the inhibition of the glucagon release leads to the following: increase of insulin secretion, decrease of gastric emptying and decrease of blood glucose levels.

Omarigliptin is for example suitable for the treatment of type 2 diabetes, obesity and high blood pressure, in particular for the treatment of type 2 diabetes.

The active pharmaceutical ingredient omarigliptin and its synthesis are described in WO 2010/056708 A1. Furthermore, WO 2013/003249 A1 describes polymorphic forms of omarigliptin free base designated forms I, II, III and IV.

WO 2015/031228 A1 discloses oral pharmaceutical compositions containing omarigliptin and pharmaceutically acceptable salts thereof respectively. According to the application "the term pharmaceutically acceptable salt refers to salts prepared from pharmaceutically acceptable non-toxic bases or acids including inorganic or organic bases and inorganic or organic acids." The application further states that "salts of basic compounds [...] are generally prepared by reacting the free base with a suitable organic or inorganic acid." The application provides a huge list of representative salts (see WO 2015/031228 A1 page 4, lines 6-21).

According to the description of WO 2015/031228 A1 "one concern with developing an oral pharmaceutical formulation of omarigliptin is the chemical stability of the compound" as "omarigliptin is sensitive to oxidation, hydrolysis (acid and base catalysed) and Maillard degradation (browning) with reducible sugar impurities in excipients" (see WO 2015/031228 A1 page 3, lines 8-10). Special emphasis is put on the acid and base catalysed hydrolysis pathway shown below (see also WO 2015/031228 A1, page 5, lines 24-37):

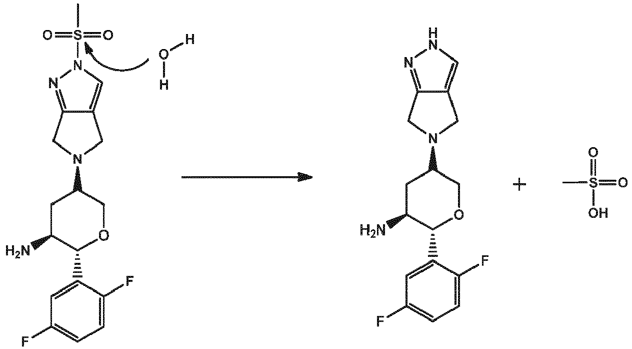

In example 4 on page 14 of WO 2015/031228 A1 pharmaceutical formulations containing the fumarate, phosphate and hydrochloride salts of omarigliptin as well as pharmaceutical formulations containing form I of the free base were tested under various conditions for stability. WO 2015/031228 A1 does neither give any information on how these salts have been prepared nor in which solid form they were present, but only teaches in a very general manner that salts of omarigliptin may be prepared by reacting omarigliptin with a suitable organic or inorganic acid. All compounds showed an increase in total degradation products, whereat increase in impurities was highest when the samples were subjected to moisture stress at 50 °C and 75% relative humidity. Strikingly, all omarigliptin salts were chemically less stable than form I of the free base.

However, salt formation is a well-established means for improving the solubility and dissolution rate of a low soluble compound like omarigliptin.
However, the so far investigated omarigliptin hydrochloride, fumarate and phosphate salt disclosed in WO 2015/031228 A1 suffer from poor chemical stability.

It is thus an objective of the present invention to provide pharmaceutically acceptable salts of omarigliptin which possess improved chemical stability, in particular improved chemical stability upon contact with moisture. A further objective of the invention was to provide pharmaceutical compositions comprising an effective amount of a chemically stable omarigliptin salt and at least one pharmaceutically acceptable excipient, which can be used in the treatment of conditions selected from the group of diabetes type 2, obesity and high blood pressure.

### SUMMARY OF THE INVENTION

The present invention solves the aforementioned problem by providing non-hygroscopic acid addition salts of omarigliptin. In particular, the invention provides crystalline non-hygroscopic acid addition salts of omarigliptin such as but not limited to omarigliptin hydrochloride, omarigliptin fumarate, omarigliptin *R*-mandelate, omarigliptin *S*-mandelate and omarigliptin *RS*-mandelate. Preferably, the present invention solves the aforementioned problem by the provision of a crystalline form of omarigliptin hydrochloride, characterized by having a powder X-ray diffractogram comprising reflections at 2-Theta angles of (18.7 ± 0.2)°, (21.0 ± 0.2)° and (24.2 ± 0.2)°, a crystalline form of omarigliptin fumarate, characterized by having a powder X-ray diffractogram comprising reflections at 2-Theta angles of (13.8 ± 0.2)°, (19.7 ± 0.2)° and (24.8 ± 0.2)°, a crystalline form of omarigliptin *R*-mandelate characterized by having a powder X-ray diffractogram at 2-Theta angles of (6.3 ± 0.2)°, (17.9 ± 0.2)° and (21.9 ± 0.2)°, a crystalline form of omarigliptin *S*-mandelate characterized by having a powder X-ray diffractogram at 2-Theta angles of (16.3 ± 0.2)°, (18.6 ± 0.2)° and (20.7 ± 0.2)° and a crystalline form of omarigliptin *RS*-mandelate characterized by having a powder X-ray diffractogram at 2-Theta angles of (6.3 ± 0.2)°, (12.7 ± 0.2)° and (18.6 ± 0.2)°.

The invention further relates to a process for the preparation of the non-hygroscopic omarigliptin acid addition salts of the present invention, including crystalline, non-hygroscopic omarigliptin acid addition salts. In addition, the invention relates to the use of the omarigliptin salts of the present invention in and/ or for the preparation of a pharmaceutical composition. The invention further relates to a pharmaceutical composition comprising an effective amount of an omarigliptin acid addition salt of the present invention.

### Abbreviations

- RT: room temperature
- RH: relative humidity
- PXRD: powder X-ray diffraction
- FTIR: Fourier transform infrared spectroscopy
- DSC: differential scanning calorimetry
- TGA: thermogravimetric analysis
- GMS: gravimetric moisture sorption

### Definitions

In the context of the present invention the following definitions have the indicated meaning, unless explicitly stated otherwise:
As used herein the term "room temperature" refers to a temperature in the range of from 20 to 30 °C, preferably to a temperature in the range of from 22 to 27°C, more preferably to a temperature in the range of from 23 to 26°C.

As used herein, the term "*R*-mandelate" refers to the *R*-mandelic acid addition salt a compound. Thus, when applied to omarigliptin, the term "*R*-mandelate" refers to the *R*-mandelic acid addition salt of omarigliptin, *R*-mandelic acid having the following structure:

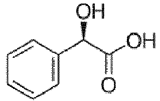

As used herein, the term "*S*-mandelate" refers to the *S*-mandelic acid addition salt a compound. Thus, when applied to omarigliptin, the term "*S*-mandelate" refers to the *S*-mandelic acid addition salt of omarigliptin, *S*-mandelic acid having the following structure:

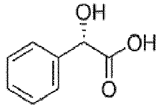

As used herein, the term "*RS*-mandelate" refers to the *RS*-mandelic acid addition salt a compound. Thus, when applied to omarigliptin, the term "*RS*-mandelate" refers to the *RS-*mandelic acid addition salt of omarigliptin, *RS*-mandelic acid being a mixture of the two enantiomers of mandelic acid, i.e. *R*-mandelic acid and *S*-mandelic acid as described above, preferably a mixture of the two *R-* and S- enantiomers in an enantiomeric ratio of from 0.25 : 0.75 to 0.75 : 0.25, more preferably in an enantiomeric ratio of from 0.45 : 0.55 to 0.55 : 0.45, even more preferably in an enantiomeric ratio of 0.5 : 0.5.

As used herein, the term "fumarate" refers to the fumaric acid addition salt a compound. Thus, when applied to omarigliptin, the term "fumarate" refers to the fumaric acid acid addition salt of omarigliptin, fumaric acid having the following structure:

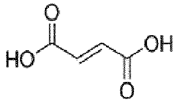

The term "non-hygroscopic" as used herein refers to a compound which shows a water uptake of at most 2.0 weight% , based on the weight of the crystalline acid addition salt, when measured with gravimetric moisture sorption at a relative humidity in the range of from 0 to 95% and a temperature of 25.0 ± 0.1 °C.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 100 to 1000 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in reflection positions and relative intensities of the reflections are to be taken into account. For example, a typical precision of the 2-theta values is in the range of ± 0.2° 2-theta, preferably in the range of ± 0.1° 2-theta. Thus, a reflection that usually appears at 3.2° 2-theta for example can appear between 3.0° and 3.4° 2-theta, preferably between 3.1 and 3.3° 2-theta on most X-ray diffractometers under standard conditions. Furthermore, one skilled in the art will appreciate that relative reflection intensities will show inter-apparatus variability as well as variability due to degree of crystallinity, preferred orientation, sample preparation and other factors known to those skilled in the art and should be taken as qualitative measure only.

The term "non-solvated" as used herein refers to a solid, where no organic solvent is coordinated in or accommodated by the crystal structure. However, non-solvated physical forms may still comprise residual organic solvents due to surface adsorption, solvent inclusions and/ or absorption in disordered regions.

The term "anhydrous" as used herein, refers to a solid, where no water is coordinated in or accommodated by the crystal structure. However, an anhydrate may still comprise residual water due to surface adsorption, solvent inclusions and/ or absorption in disordered regions.

As used herein, the term "mother liquor" refers to the solution remaining after crystallization of a solid.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

### BRIEF DESCRIPTION OF THE DRAWINGS

**Figure 1****:** illustrates a representative PXRD of crystalline omarigliptin hydrochloride of the present invention. The x-axis shows the scattering angle in °2-Theta, the y-axis shows the intensity of the scattered X-ray beam in counts of detected photons.
**Figure 2****:** illustrates a representative FTIR spectrum of crystalline omarigliptin hydrochloride of the present invention. The x-axis shows the mid-IR region in cm⁻¹ (reciprocal centimeters, also called wave numbers), the y-axis shows the transmittance in percent (%).
**Figure 3****:** illustrates a representative DSC curve of crystalline omarigliptin hydrochloride of the present invention. The x-axis shows the temperature in degree Celsius (°C), the y-axis shows the heat flow rate in W/g (with endothermic peaks going up).
**Figure 4****:** illustrates a representative TGA curve of crystalline omarigliptin hydrochloride of the present invention. The x-axis shows the temperature in degree Celsius (°C), the y-axis shows the mass (loss) of the sample in percent (%).
**Figure 5****:** illustrates a representative PXRD of crystalline omarigliptin fumarate of the present invention. The x-axis shows the scattering angle in °2-Theta, the y-axis shows the intensity of the scattered X-ray beam in counts of detected photons.
**Figure 6****:** illustrates a representative FTIR spectrum of crystalline omarigliptin fumarate of the present invention. The x-axis shows the mid-IR region in cm⁻¹ (reciprocal centimeters, also called wave numbers), the y-axis shows the transmittance in percent (%).
**Figure 7****:** illustrates a representative DSC curve of crystalline omarigliptin fumarate of the present invention. The x-axis shows the temperature in degree Celsius (°C), the y-axis shows the heat flow rate in W/g (with endothermic peaks going up).
**Figure 8****:** illustrates a representative TGA curve of crystalline omarigliptin fumarate of the present invention. The x-axis shows the temperature in degree Celsius (°C), the y-axis shows the mass (loss) of the sample in percent (%).
**Figure 9****:** illustrates a representative PXRD of crystalline omarigliptin *R*-mandelate of the present invention. The x-axis shows the scattering angle in °2-Theta, the y-axis shows the intensity of the scattered X-ray beam in counts of detected photons.
**Figure 10****:** illustrates a representative FTIR spectrum of crystalline omarigliptin *R*-mandelate of the present invention. The x-axis shows the mid-IR region in cm⁻¹ (reciprocal centimeters, also called wave numbers), the y-axis shows the transmittance in percent (%).
**Figure 11****:** illustrates a representative DSC curve of crystalline omarigliptin *R*-mandelate of the present invention. The x-axis shows the temperature in degree Celsius (°C), the y-axis shows the heat flow rate in W/g (with endothermic peaks going up).
**Figure 12****:** illustrates a representative TGA curve of crystalline omarigliptin *R*-mandelate of the present invention. The x-axis shows the temperature in degree Celsius (°C), the y-axis shows the mass (loss) of the sample in percent (%).
**Figure 13****:** illustrates a representative PXRD of crystalline omarigliptin *S*-mandelate of the present invention. The x-axis shows the scattering angle in °2-Theta, the y-axis shows the intensity of the scattered X-ray beam in counts of detected photons.
**Figure 14****:** illustrates a representative FTIR spectrum of crystalline omarigliptin *S*-mandelate of the present invention. The x-axis shows the mid-IR region in cm⁻¹ (reciprocal centimeters, also called wave numbers), the y-axis shows the transmittance in percent (%).
**Figure 15****:** illustrates a representative DSC curve of crystalline omarigliptin *S*-mandelate of the present invention. The x-axis shows the temperature in degree Celsius (°C), the y-axis shows the heat flow rate in W/g (with endothermic peaks going up).
**Figure 16****:** illustrates a representative TGA curve of crystalline omarigliptin *S*-mandelate of the present invention. The x-axis shows the temperature in degree Celsius (°C), the y-axis shows the mass (loss) of the sample in percent (%).
**Figure 17****:** illustrates a representative PXRD of crystalline omarigliptin *RS*-mandelate of the present invention. The x-axis shows the scattering angle in °2-Theta, the y-axis shows the intensity of the scattered X-ray beam in counts of detected photons.
**Figure 18****:** illustrates a representative FTIR spectrum of crystalline omarigliptin *RS*-mandelate of the present invention. The x-axis shows the mid-IR region in cm⁻¹ (reciprocal centimeters, also called wave numbers), the y-axis shows the transmittance in percent (%).
**Figure 19****:** illustrates a representative DSC curve of crystalline omarigliptin *RS*-mandelate of the present invention. The x-axis shows the temperature in degree Celsius (°C), the y-axis shows the heat flow rate in W/g (with endothermic peaks going up).
**Figure 20****:** illustrates a representative TGA curve of crystalline omarigliptin *RS*-mandelate of the present invention. The x-axis shows the temperature in degree Celsius (°C), the y-axis shows the mass (loss) of the sample in percent (%).

### DETAILED DESCRIPTION OF THE INVENTION

The present invention provides non-hygroscopic acid addition salts of omarigliptin.

The non-hygroscopic omarigliptin acid addition salts of the invention are characterized by high chemical stability, in particular against hydrolysis and high physical stability even when subjected to severe moisture stress.

Their excellent stabilities enable the omarigliptin acid addition salts of the present invention to be used in and for the preparation of a pharmaceutical composition, in particular a pharmaceutical composition for the treatment of diabetes mellitus type 2, obesity and high blood pressure. Due to their non-hygroscopic nature and consequently improved stability, especially against hydrolysis there are hardly any restrictions with regards to the use of excipients, e.g. there is no need to mainly use neutral and/ or dry excipients, which advantageously enlarges the repertoire of formulation scientists, when formulating omarigliptin salts of the present invention. In addition, due to their superior stability properties the non-hygroscopic omarigliptin salts of the invention are favourable physical forms for storage and shipping.

In one aspect, the present invention relates to an acid addition salt of omarigliptin, wherein the omarigliptin acid addition salt shows a water uptake of at most 2.0 weight%, preferably of at most 1.4 weight%, more preferably of at most 1.0 weight%, even more preferably of at most 0.8 weight% and most preferably of at most 0.7 weight%, based on the weight of the omarigliptin acid addition salt, when measured with gravimetric moisture sorption at a relative humidity in the range of from 0 to 95% and a temperature of 25.0 ± 0.1 °C. Preferably, the omarigliptin acid addition salt of the invention is crystalline.

Alternatively or additionally, the omarigliptin acid addition salt of the invention is characterized by showing a weight loss of about 0.5 weight% or less, preferably of about 0.4 weight% or less, more preferably of about 0.3 weight% or less, even more preferably of about 0.2 weight% or less and most preferably of about 0.1 weight% or less based on the weight of the omarigliptin acid addition salt, when measured with TGA at a temperature in the range of from about 25 to 200 °C and a heating rate of about 10 K/min. In a preferred embodiment the omarigliptin acid addition salt of the invention is non-solvated.

In a preferred embodiment, the omarigliptin acid addition salt of the invention is selected from the group consisting of omarigliptin hydrochloride, omarigliptin fumarate, omarigliptin R-mandelate, omarigliptin *S*-mandelate and omarigliptin *RS-*mandelate.

In a particular embodiment, the present invention relates to crystalline omarigliptin hydrochloride characterized by having a PXRD comprising reflections at 2-Theta angles selected from the group of
(a) (18.7 ± 0.2)°, (21.0 ± 0.2)° and (24.2 ± 0.2)°,
(b) (10.1 ± 0.2)°, (18.7 ± 0.2)°, (21.0 ± 0.2)° and (24.2 ± 0.2)°,
(c) (8.7 ± 0.2)°, (10.1 ± 0.2)°, (18.7 ± 0.2)°, (21.0 ± 0.2)° and (24.2 ± 0.2)°,
(d) (4.8 ± 0.2)°, (8.7 ± 0.2)°, (10.1 ± 0.2)°, (18.7 ± 0.2)°, (21.0 ± 0.2)° and (24.2 ± 0.2)°,
(e) (4.8 ± 0.2)°, (8.7 ± 0.2)°, (10.1 ± 0.2)°, (16.5 ± 0.2)°, (18.7 ± 0.2)°, (21.0 ± 0.2)° and (24.2 ± 0.2)°,
(f) (4.8 ± 0.2)°, (8.7 ± 0.2)°, (10.1 ± 0.2)°, (16.5 ± 0.2)°, (17.4 ± 0.2)°, (18.7 ± 0.2)°, (21.0 ± 0.2)° and (24.2 ± 0.2)°,
(g) (4.8 ± 0.2)°, (8.7 ± 0.2)°, (10.1 ± 0.2)°, (16.5 ± 0.2)°, (17.4 ± 0.2)°, (18.7 ± 0.2)°, (20.3 ± 0.2)°, (21.0 ± 0.2)° and (24.2 ± 0.2)° or
(h) (4.8 ± 0.2)°, (8.7 ± 0.2)°, (10.1 ± 0.2)°, (16.5 ± 0.2)°, (17.4 ± 0.2)°, (18.7 ± 0.2)°, (20.3 ± 0.2)°, (20.8 ± 0.2)°, (21.0 ± 0.2)° and (24.2 ± 0.2)°,
when measured with Cu-Kalpha_{1,2} radiation at a wavelength of 0.15419 nm and room temperature.

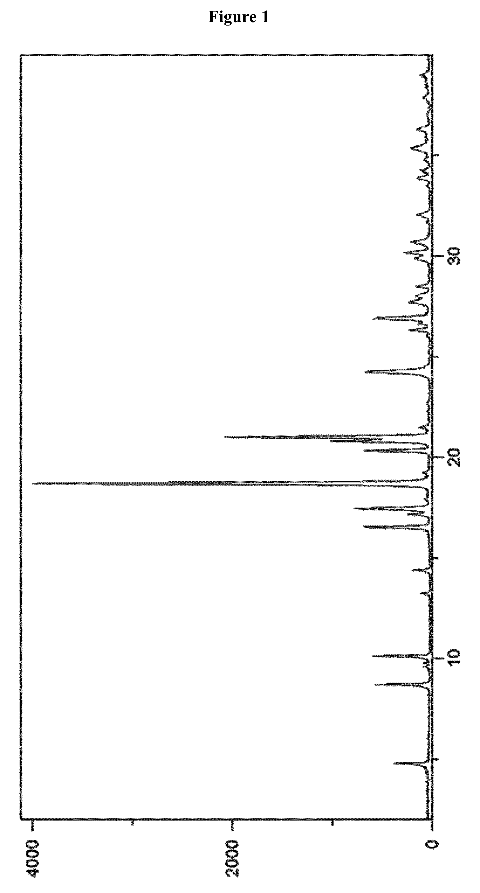

Alternatively, crystalline omarigliptin hydrochloride of the invention is characterized by having a PXRD essentially the same as displayed in figure 1 of the present invention, when measured with Cu-Kalpha_{1,2} radiation at a wavelength of 0.15419 nm and room temperature.

Alternatively or additionally, crystalline omarigliptin hydrochloride is characterized by having a FTIR spectrum comprising peaks at wavenumbers of (2849 ± 2) cm⁻¹, (2794 ± 2) cm⁻¹, (1515 ± 2) cm⁻¹, (1326 ± 2) cm⁻¹ and (1274 ± 2) cm⁻¹, when measured with a diamond ATR cell at room temperature.

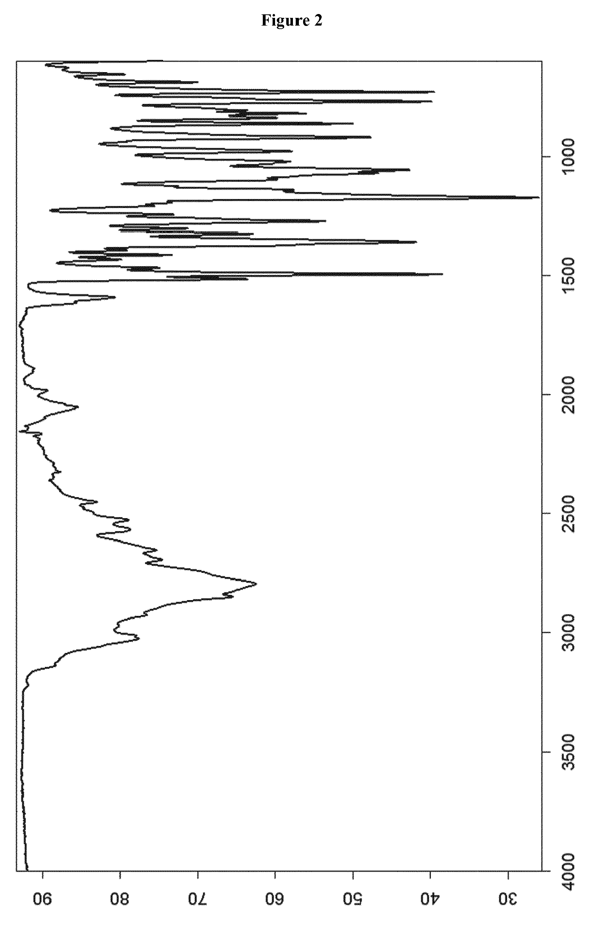

Alternatively, crystalline omarigliptin hydrochloride of the invention is characterized by having a FTIR spectrum essentially the same as displayed in figure 2 of the present invention, when measured with a diamond ATR cell at room temperature.

Alternatively or additionally, crystalline omarigliptin hydrochloride of the invention is characterized by having a DSC curve showing no thermal event up to a temperature of about 200 °C, when measured at a heating rate of 10 K/min. Preferably, crystalline omarigliptin hydrochloride of the invention is characterized by exhibiting no melting point.

Alternatively or additionally, crystalline omarigliptin hydrochloride of the invention is characterized by showing a weight loss of about 0.5 weight% or less, preferably of about 0.4 weight% or less, more preferably of about 0.3 weight% or less, even more preferably of about 0.2 weight% or less and most preferably of about 0.1 weight% or less based on the weight of crystalline omarigliptin hydrochloride, when measured with TGA at a temperature in the range of from about 25 to 200 °C and a heating rate of about 10 K/min. In a preferred embodiment crystalline omarigliptin hydrochloride of the invention is non-solvated.

Preferably, crystalline omarigliptin hydrochloride of the invention is characterized by showing a water uptake of at most 2.0 weight% based on the weight of crystalline omarigliptin hydrochloride, when measured with GMS from about 0 to 95% relative humidity at a temperature of 25.0 ± 0.1 °C. In a preferred embodiment crystalline omarigliptin hydrochloride of the invention is non-hygroscopic.

In another preferred embodiment, the present invention relates to crystalline omarigliptin fumarate characterized by having a PXRD comprising reflections at 2-Theta angles selected from the group of
(a) (13.8 ± 0.2)°, (19.7 ± 0.2)° and (21.2 ± 0.2)°,
(b) (11.7 ± 0.2)°, (13.8 ± 0.2)°, (19.7 ± 0.2)° and (21.2 ± 0.2)°,
(c) (6.9 ± 0.2)°, (11.7 ± 0.2)°, (13.8 ± 0.2)°, (19.7 ± 0.2)° and (21.2 ± 0.2)°,
(d) (6.9 ± 0.2)°, (8.9 ± 0.2)°, (11.7 ± 0.2)°, (13.8 ± 0.2)°, (19.7 ± 0.2)° and (21.2 ± 0.2)°,
(e) (6.9 ± 0.2)°, (8.9 ± 0.2)°, (10.8 ± 0.2)°, (11.7 ± 0.2)°, (13.8 ± 0.2)°, (19.7 ± 0.2)° and (21.2 ± 0.2)°,
(f) (6.9 ± 0.2)°, (8.9 ± 0.2)°, (10.8 ± 0.2)°, (11.7 ± 0.2)°, (13.8 ± 0.2)°, (19.7 ± 0.2)°, (21.2 ± 0.2)° and (22.1 ± 0.2)°,
(g) (6.9 ± 0.2)°, (8.9 ± 0.2)°, (10.8 ± 0.2)°, (11.7 ± 0.2)°, (13.8 ± 0.2)°, (19.7 ± 0.2)°, (21.2 ± 0.2)°, (22.1 ± 0.2)° and (22.5 ± 0.2)° or
(h) (6.9 ± 0.2)°, (8.9 ± 0.2)°, (10.8 ± 0.2)°, (11.7 ± 0.2)°, (13.8 ± 0.2)°, (19.7 ± 0.2)°, (21.2 ± 0.2)°, (22.1 ± 0.2)°, (22.5 ± 0.2)° and (24.8 ± 0.2)°,
when measured with Cu-Kalpha_{1,2} radiation at a wavelength of 0.15419 nm and room temperature.

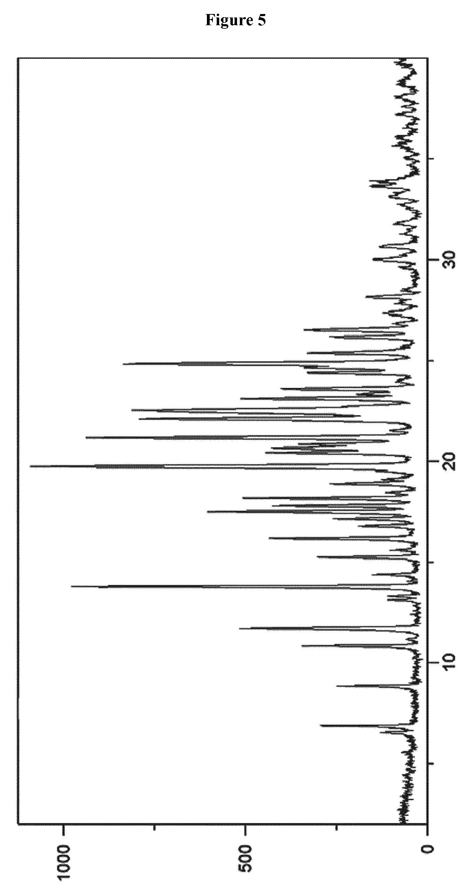

Alternatively, crystalline omarigliptin fumarate of the invention is characterized by having a PXRD essentially the same as displayed in figure 5 of the present invention, when measured with Cu-Kalpha_{1,2} radiation at a wavelength of 0.15419 nm and room temperature.

Alternatively or additionally, crystalline omarigliptin fumarate is characterized by having a FTIR spectrum comprising peaks at wavenumbers of (3301 ± 2) cm⁻¹, (3088 ± 2) cm⁻¹, (1573 ± 2) cm⁻¹, (1359 ± 2) cm⁻¹ and (1165 ± 2) cm⁻¹, when measured with a diamond ATR cell room temperature.

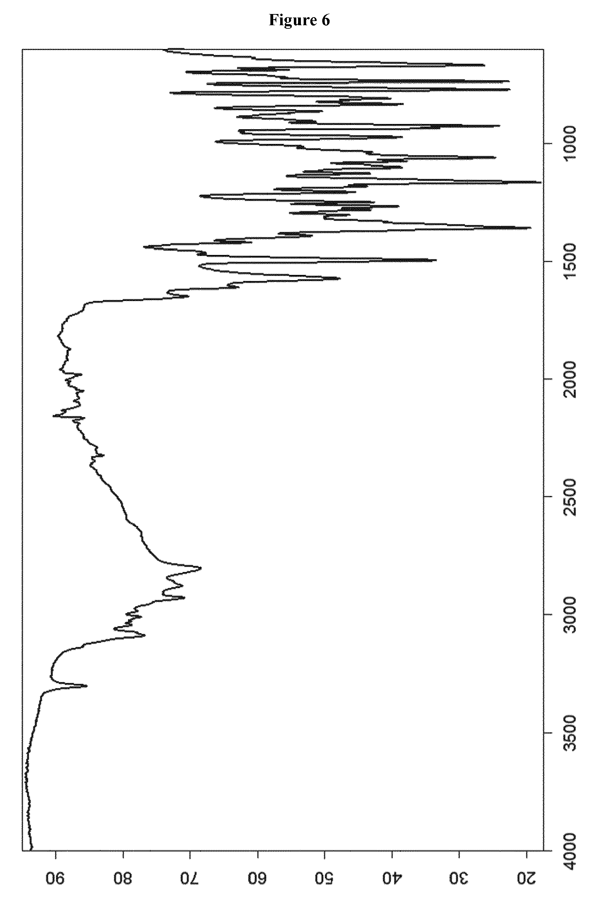

Alternatively, crystalline omarigliptin fumarate of the invention is characterized by having a FTIR spectrum essentially the same as displayed in figure 6 of the present invention, when measured with a diamond ATR cell at room temperature.

Alternatively or additionally, crystalline omarigliptin fumarate of the invention is characterized by having a DSC curve showing no thermal event up to a temperature of about 170 °C, when measured at a heating rate of 10 K/min. Preferably, crystalline omarigliptin fumarate of the invention is characterized by having a melting point at a temperature in the range of from about 175 to 201 °C, when measured with DSC at a heating rate of 10 K/min.

Alternatively or additionally, crystalline omarigliptin fumarate of the invention is characterized by showing a weight loss of about 0.5 weight% or less, preferably of about 0.4 weight% or less, more preferably of about 0.3 weight% or less, even more preferably of about 0.2 weight% or less and most preferably of about 0.1 weight % or less based on the weight of the crystalline omarigliptin fumarate of the present invention, when measured with TGA at a temperature in the range of from about 25 to 200 °C and a heating rate of about 10 K/min. In a preferred embodiment crystalline omarigliptin fumarate of the invention is non-solvated.

Preferably, crystalline omarigliptin fumarate of the invention is characterized by showing a water uptake of at most 2.0 weight%, preferably of at most 1.5 weight% based on the weight of crystalline omarigliptin fumarate of the present invention, when measured with GMS from about 0 to 95% relative humidity at a temperature of 25.0 ± 0.1 °C. In a preferred embodiment crystalline omarigliptin fumarate of the invention is non-hygroscopic.

In another preferred embodiment the present invention relates to crystalline omarigliptin R-mandelate characterized by having a PXRD comprising reflections at 2-Theta angles selected from the group of
(a) (6.3 ± 0.2)°, (17.9 ± 0.2)° and (21.9 ± 0.2)°,
(b) (6.3 ± 0.2)°, (17.9 ± 0.2)°, (21.9 ± 0.2)° and (28.0 ± 0.2)°,
(c) (6.3 ± 0.2)°, (12.6± 0.2)°, (17.9 ± 0.2)°, (21.9 ± 0.2)° and (28.0 ± 0.2)°,
(d) (6.3 ± 0.2)°, (7.1 ± 0.2)°, (12.6 ± 0.2)°, (17.9 ± 0.2)°, (21.9 ± 0.2)° and (28.0 ± 0.2)°,
(e) (6.3 ± 0.2)°, (7.1 ± 0.2)°, (12.6 ± 0.2)°, (15.1 ± 0.2)°, (17.9 ± 0.2)°, (21.9 ± 0.2)° and (28.0 ± 0.2)°,
(f) (6.3 ± 0.2)°, (7.1 ± 0.2)°, (12.6 ± 0.2)°, (15.1 ± 0.2)°, (15.3 ± 0.2)°, (17.9 ± 0.2)°, (21.9 ± 0.2)° and (28.0 ± 0.2)°,
(g) (6.3 ± 0.2)°, (7.1 ± 0.2)°, (12.6 ± 0.2)°, (15.1 ± 0.2)°, (15.3 ± 0.2)°, (17.9 ± 0.2)°, (20.8 ± 0.2)°, (21.9 ± 0.2)° and (28.0 ± 0.2)° or
(h) (6.3± 0.2)°, (7.1 ± 0.2)°, (12.6 ± 0.2)°, (15.1 ± 0.2)°, (15.3 ± 0.2)°, (17.9 ± 0.2)°, (20.8 ± 0.2)°, (21.9 ± 0.2)°, (23.2 ± 0.2)° and (28.0 ± 0.2)°,
when measured with Cu-Kalpha_{1,2} radiation at a wavelength of 0.15419 nm and room temperature.

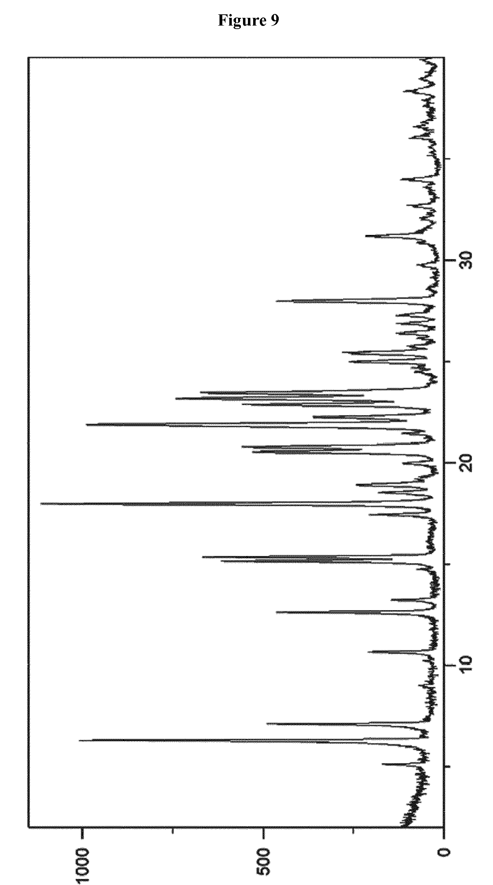

Alternatively, crystalline omarigliptin *R*-mandelate of the invention is characterized by having a PXRD essentially the same as displayed in figure 9 of the present invention, when measured with Cu-Kalpha_{1,2} radiation at a wavelength of 0.15419 nm and room temperature.

Alternatively or additionally, crystalline omarigliptin *R*-mandelate is characterized by having a FTIR spectrum comprising peaks at wavenumbers of (3390 ± 2) cm⁻¹, (1636 ± 2) cm⁻¹, (1487 ± 2) cm⁻¹ (1371 ± 2) cm⁻¹ and (1037 ± 2) cm⁻¹, when measured with a diamond ATR cell at room temperature.

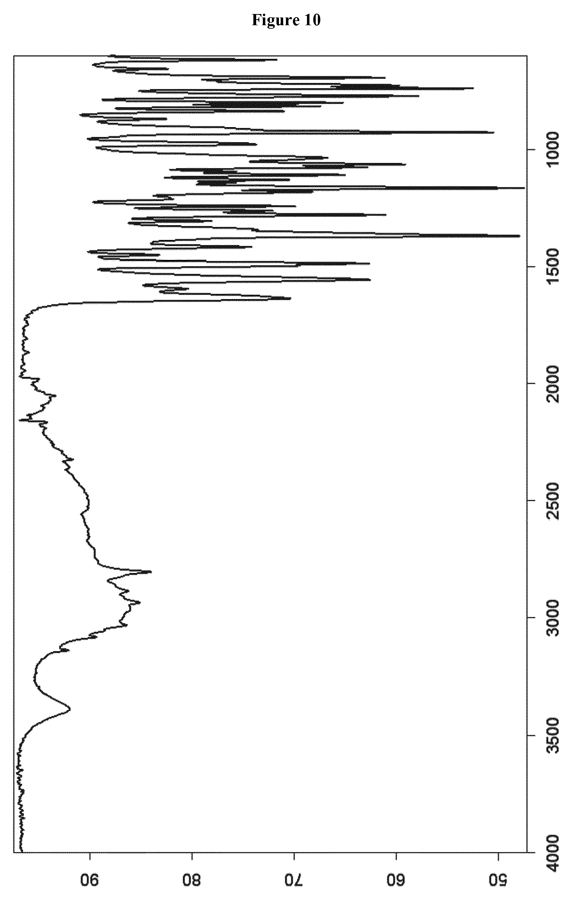

Alternatively, crystalline omarigliptin *R*-mandelate of the invention is characterized by having a FTIR spectrum essentially the same as displayed in figure 10 of the present invention, when measured with a diamond ATR cell at room temperature.

Alternatively or additionally, crystalline omarigliptin *R*-mandelate of the invention is characterized by having a DSC curve showing no thermal event up to a temperature of about 200 °C, when measured at a heating rate of 10 K/min. Preferably, crystalline omarigliptin R-mandelate of the invention is characterized by having a melting point at a temperature in the range of from about 204 to 206 °C, when measured with DSC at a heating rate of 10 K/min.

Alternatively or additionally, crystalline omarigliptin *R*-mandelate of the invention is characterized by showing a weight loss of about 0.5 weight% or less, preferably of about 0.4 weight% or less, more preferably of about 0.3 weight% or less, even more preferably of about 0.2 weight% or less and most preferably of about 0.1 weight % or less, based on the weight of the crystalline omarigliptin *R*-mandelate of the present invention, when measured with TGA at a temperature in the range of from about 25 to 200 °C and a heating rate of about 10 K/min. In a preferred embodiment crystalline omarigliptin *R*-mandelate of the invention is non-solvated.

Preferably, crystalline omarigliptin *R*-mandelate of the invention is characterized by showing a water uptake of at most 2.0 weight%, preferably of at most 1.5 weight%, more preferably of at most 1.0 weight% and most preferably of at most 0.8 weight% or less, based on the weight of crystalline omarigliptin *R*-mandelate of the present invention, when measured with GMS from about 0 to 95% relative humidity at a temperature of 25.0 ± 0.1 °C. In a preferred embodiment crystalline omarigliptin *R*-mandelate of the invention is non-hygroscopic.

In another preferred embodiment, the present invention relates to crystalline omarigliptin *S-*mandelate characterized by having a PXRD comprising reflections at 2-Theta angles selected from the group of
(a) (16.3 ± 0.2)°, (18.6 ± 0.2)° and (20.7 ± 0.2)°,
(b) (7.1 ± 0.2)°, (16.3 ± 0.2)°, (18.6 ± 0.2)° and (20.7 ± 0.2)°,
(c) (7.1 ± 0.2)°, (8.8± 0.2)°, (16.3 ± 0.2)°, (18.6 ± 0.2)° and (20.7 ± 0.2)°,
(d) (7.1 ± 0.2)°, (8.8 ± 0.2)°, (16.3 ± 0.2)°, (18.2 ± 0.2)°, (18.6 ± 0.2)° and (20.7 ± 0.2)°,
(e) (7.1 ± 0.2)°, (8.8 ± 0.2)°, (16.3 ± 0.2)°, (17.4 ± 0.2)°, (18.2 ± 0.2)°, (18.6 ± 0.2)° and (20.7± 0.2)°,
(f) (7.1 ± 0.2)°, (8.8 ± 0.2)°, (16.3 ± 0.2)°, (17.4 ± 0.2)°, (18.2 ± 0.2)°, (18.6 ± 0.2)°, (20.7 ± 0.2)° and (21.0 ± 0.2)°,
(g) (7.1 ± 0.2)°, (8.8 ± 0.2)°, (16.3 ± 0.2)°, (17.4 ± 0.2)°, (18.2 ± 0.2)°, (18.6 ± 0.2)°, (20.7 ± 0.2)°, (21.0 ± 0.2)° and (22.8 ± 0.2)° or
(h) (7.1 ± 0.2)°, (8.8 ± 0.2)°, (14.9 ± 0.2)°, (16.3 ± 0.2)°, (17.4 ± 0.2)°, (18.2 ± 0.2)°, (18.6 ± 0.2)°, (20.7 ± 0.2)°, (21.0 ± 0.2)° and (22.8 ± 0.2)°,
when measured with Cu-Kalpha_{1,2} radiation at a wavelength of 0.15419 nm and room temperature.

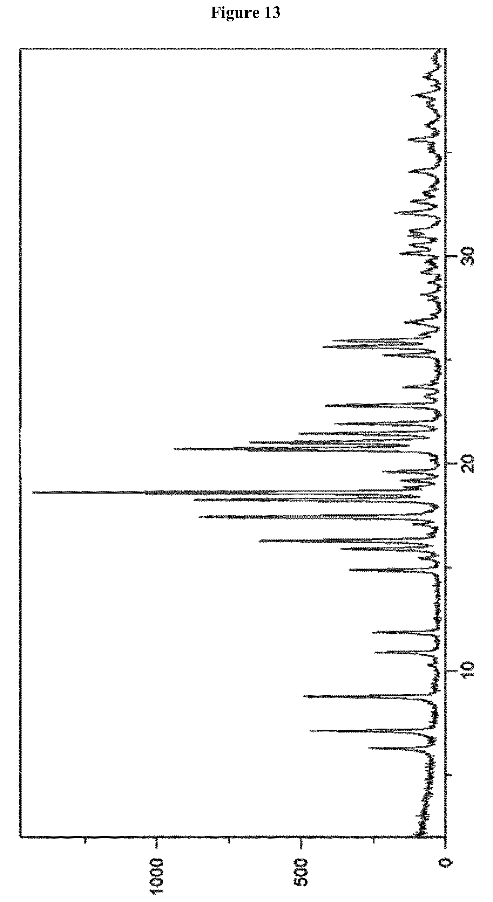

Alternatively, crystalline omarigliptin *S*-mandelate of the invention is characterized by having a PXRD essentially the same as displayed in figure 13 of the present invention, when measured with Cu-Kalpha_{1,2} radiation at a wavelength of 0.15419 nm and room temperature.

Alternatively or additionally, crystalline omarigliptin *S*-mandelate is characterized by having a FTIR spectrum comprising peaks at wavenumbers of (3292 ± 2) cm⁻¹, (1544 ± 2) cm⁻¹, (1432 ± 2) cm⁻¹, (1365 ± 2) cm⁻¹ and (1218 ± 2) cm⁻¹, when measured with a diamond ATR cell at room temperature.

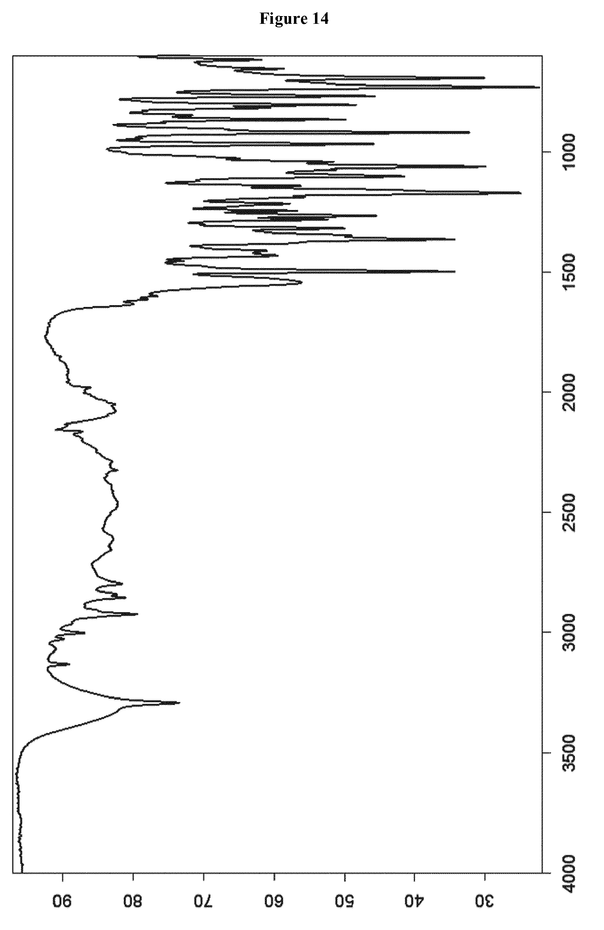

Alternatively, crystalline omarigliptin *S*-mandelate of the invention is characterized by having a FTIR spectrum essentially the same as displayed in figure 14 of the present invention, when measured with a diamond ATR cell at room temperature.

Alternatively or additionally, crystalline omarigliptin *S*-mandelate of the invention is characterized by having a DSC curve showing no thermal event up to a temperature of about 180 °C, when measured at a heating rate of 10 K/min. Preferably, crystalline omarigliptin *S-*mandelate of the invention is characterized by having a melting point at a temperature in the range of from about 185 to 187 °C, when measured with DSC at a heating rate of 10 K/min.

Alternatively or additionally, crystalline omarigliptin *S*-mandelate of the invention is characterized by showing a weight loss of about 0.5 weight% or less, preferably of about 0.4 weight% or less, more preferably of about 0.3 weight% or less, even more preferably of about 0.2 weight% or less and most preferably of about 0.1 weight % or less, based on the weight of the crystalline omarigliptin *S*-mandelate of the present invention, when measured with TGA at a temperature in the range of from about 25 to 200 °C and a heating rate of about 10 K/min. In a preferred embodiment crystalline omarigliptin *S*-mandelate of the invention is non-solvated.

Preferably, crystalline omarigliptin *S*-mandelate of the invention is characterized by showing a water uptake of at most 2.0 weight%, preferably of at most 1.5 weight%, more preferably of at most 1.0 weight%, based on the weight of crystalline omarigliptin *S*-mandelate of the present invention, when measured with GMS from about 0 to 95% relative humidity at a temperature of 25.0 ± 0.1 °C. In a preferred embodiment crystalline omarigliptin *S*-mandelate of the invention is non-hygroscopic.

In another preferred embodiment, the present invention relates to crystalline omarigliptin *RS-*mandelate characterized by having a PXRD comprising reflections at 2-Theta angles selected from the group of
(a) (6.3 ± 0.2)°, (12.7 ± 0.2)° and (18.6 ± 0.2)°,
(b) (6.3 ± 0.2)°, (7.1 ± 0.2)°, (12.7 ± 0.2)° and (18.6 ± 0.2)°,
(c) (6.3 ± 0.2)°, (7.1 ± 0.2)°, (8.8 ± 0.2)°, (12.7 ± 0.2)° and (18.6 ± 0.2)°,
(d) (6.3 ± 0.2)°, (7.1 ± 0.2)°, (8.8 ± 0.2)°, (12.7 ± 0.2)°, (16.3 ± 0.2)° and (18.6 ± 0.2)°,
(e) (6.3 ± 0.2)°, (7.1 ± 0.2)°, (8.8 ± 0.2)°, (12.7 ± 0.2)°, (16.3 ± 0.2)°, (18.6 ± 0.2)° and (20.7 ± 0.2)°,
(f) (6.3 ± 0.2)°, (7.1 ± 0.2)°, (8.8 ± 0.2)°, (12.7 ± 0.2)°, (16.3 ± 0.2)°, (18.6 ± 0.2)°, (20.7 ± 0.2)° and (21.7 ± 0.2)°,
(g) (6.3 ± 0.2)°, (7.1 ± 0.2)°, (8.8 ± 0.2)°, (12.7 ± 0.2)°, (16.3 ± 0.2)°, (18.6 ± 0.2)°, (20.7 ± 0.2)°, (21.7 ± 0.2)° and (23.3 ± 0.2)° or
(h) (6.3 ± 0.2)°, (7.1 ± 0.2)°, (8.8 ± 0.2)°, (12.7 ± 0.2)°, (15.4 ± 0.2)°, (16.3 ± 0.2)°, (18.6 ± 0.2)°, (20.7 ± 0.2)°, (21.7 ± 0.2)° and (23.3 ± 0.2)°,
when measured with Cu-Kalpha_{1,2} radiation at a wavelength of 0.15419 nm at room temperature.

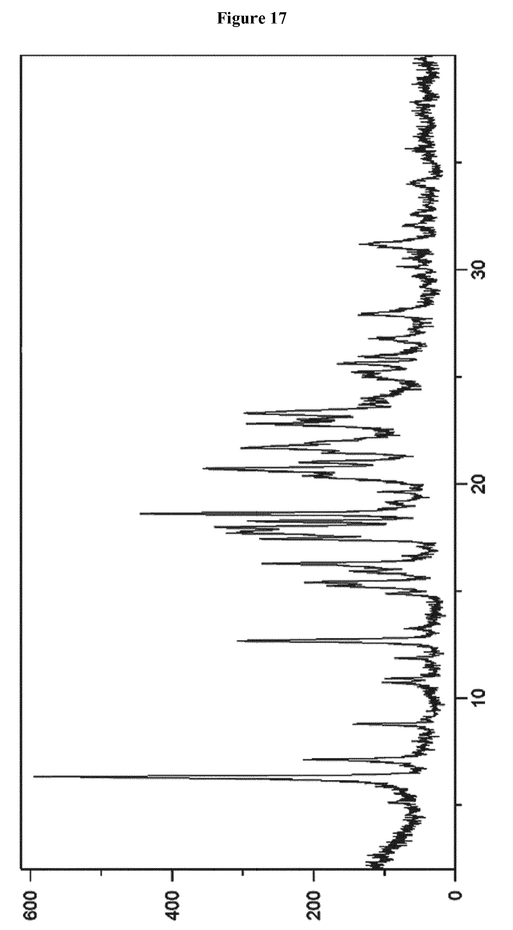

Alternatively, crystalline omarigliptin *RS*-mandelate of the invention is characterized by having a PXRD essentially the same as displayed in figure 17 of the present invention, when measured with Cu-Kalpha_{1,2} radiation at a wavelength of 0.15419 nm at room temperature.

Alternatively or additionally, crystalline omarigliptin *RS*-mandelate is characterized by having a FTIR spectrum comprising peaks at wavenumbers of (1635 ± 2) cm⁻¹, (1554 ± 2) cm⁻¹, (1367 ± 2) cm⁻¹, (1108 ± 2) cm⁻¹ and (926 ± 2) cm⁻¹, when measured with a diamond ATR cell at room temperature.

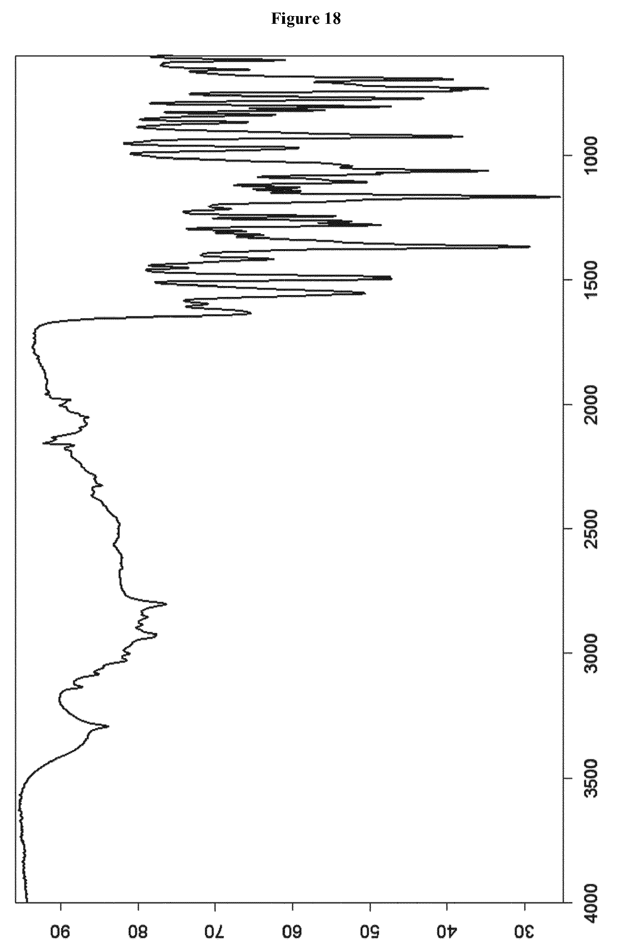

Alternatively, crystalline omarigliptin *RS*-mandelate of the invention is characterized by having a FTIR spectrum essentially the same as displayed in figure 18 of the present invention, when measured with a diamond ATR cell at room temperature.

Alternatively or additionally, crystalline omarigliptin *RS*-mandelate of the invention is characterized by having a DSC curve showing no thermal event up to a temperature of about 175 °C, when measured at a heating rate of 10 K/min. Preferably, crystalline omarigliptin *RS-*mandelate of the invention is characterized by having a first melting point at a temperature in the range of from about 175 to 183 °C and a second melting point at a temperature in the range of from about 190 to 198 °C, when measured with DSC at a heating rate of 10 K/min.

Alternatively or additionally, crystalline omarigliptin *RS*-mandelate of the invention is characterized by showing a weight loss of about 0.5 weight% or less, preferably of about 0.4 weight% or less, more preferably of about 0.3 weight% or less, even more preferably of about 0.2 weight% or less and most preferably of about 0.1 weight % or less based on the weight of the crystalline omarigliptin *RS*-mandelate of the present invention, when measured with TGA at a temperature in the range of from about 25 to 180 °C and a heating rate of about 10 K/min. In a preferred embodiment crystalline omarigliptin *RS*-mandelate of the invention is non-solvated.

Preferably, crystalline omarigliptin *RS*-mandelate of the invention is characterized by showing a water uptake of at most 2.0 weight%, preferably of at most 1.5 weight%, more preferably at most 1.0 weight% and most preferably of at most 0.8 weight%, based on the weight of the crystalline omarigliptin *RS*-mandelate of the present invention, when measured with GMS from about 0 to 95% relative humidity at a temperature of 25.0 ±0.1 °C. In a preferred embodiment crystalline omarigliptin *RS*-mandelate of the invention is non-hygroscopic.

The acid addition salts of the present invention are characterized by having a molar ratio of omarigliptin and acid in the range of from about 1.0 : 0.8 to 1.0 : 1.2, more preferably from about 1.0 : 0.9 to 1.0 : 1.1 and most preferably the molar ratio is about 1.0 to 1.0. Hence, the non-hygroscopic acid addition salts of omarigliptin, preferably the crystalline non-hygroscopic acid addition salts of omarigliptin according to the present invention are mono-acid addition salts.

In a further aspect, the present invention relates to a process for the preparation of a non-hygroscopic omarigliptin acid addition salt, preferably a crystalline, non-hygroscopic omarigliptin acid addition salt according to the present invention comprising:
(i) Providing the free base of omarigliptin in amorphous form, pseudo-amorphous form, crystalline form or as a mixture of two or more of these forms;
(ii) Preparing a mixture comprising the omarigliptin provided in step (i) and a solvent system comprising at least one alcohol;
(iii) Reacting the free base of omarigliptin comprised in the mixture provided in (ii) with an acid to obtain an acid addition salt of omarigliptin
(iv) Separating the non-hygroscopic omarigliptin acid addition salt of step (iii) from the reaction mixture.

Preferably, the non-hygroscopic acid addition salt of omarigliptin is the non-hygroscopic acid addition salt omarigliptin hydrochloride, omarigliptin fumarate, omarigliptin *R*-mandelate, omarigliptin *S-*mandelate or omarigliptin *RS-*mandelate. More preferably, the crystalline non-hygroscopic acid addition salt of omarigliptin is the crystalline, non-hygroscopic acid addition salt crystalline omarigliptin hydrochloride, crystalline omarigliptin fumarate, crystalline omarigliptin *R*-mandelate, crystalline omarigliptin *S-*mandelate or crystalline omarigliptin *RS-*mandelate.

In step (i), the free base of omarigliptin is provided. Omarigliptin free base may be prepared according to the procedure disclosed in WO 2010/056708 A1. Any form of omarigliptin may be applied as starting material for example crystalline, amorphous or a mixture of crystalline and amorphous omarigliptin can be used as starting material. Suitable crystalline forms are for example forms I to IV of WO 2013/003249 A1 or mixtures thereof, which may be prepared according to the procedures described in the same application.

In step (ii) a mixture comprising the omarigliptin free base provided in step (i) and a solvent system comprising at least one alcohol is prepared. The at least one alcohol is preferably an alcohol having 1 to 5 carbon atoms, i.e. a Cl-C5 alcohol. Preferably, the at least one alcohol is selected from the group consisting of methanol, ethanol, 1-propanol, 2-propanol, 1-butanol, 2-butanol, isobutanol, tert-butanol and isoamylalcohol, more preferably the alcohol is selected from ethanol and 2-propanol and most preferably the at least one alcohol is ethanol. The alcohol content of the solvent system is preferably in the range of from about 50 to 100 volume%, preferably from about 75 to 100 volume%, most preferably from about 90 to 100 volume%. Preferably, the solvent system comprises no additional organic or inorganic solvent. More preferably, the solvent system comprises no additional organic solvent. The solvent system may additionally comprise water in an amount in the range of from about 1 to 50 volume% (v/v), preferably from about 1 to 25 volume% and more preferably from about 1 to 10 volume%. Preferably, omarigliptin is applied at a concentration in the range of from about 5 to 50 g/L, more preferably from about 10 to 40 g/ and most preferably from about 15 to 35 g/L solvent. Preferably, preparing a mixture comprising the omarigliptin provided in step (i) and a solvent system comprising at least one alcohol according to step (ii) comprises dissolving, more preferably completely dissolving the omarigliptin free base provided in (i) in the solvent system comprising the at least one alcohol.

Thus, in step (ii) omarigliptin free base may be dissolved in the solvent system for example by heating to reflux temperature.

In step (iii), the omarigliptin free base comprised in the mixture provided in (ii) is reacted with an acid to provide an omarigliptin acid addition salt, preferably with an acid selected from the group of hydrochloric acid, fumaric acid, *R*-mandelic acid, *S*-mandelic acid and *RS-*mandelic acid e.g. by adding the acid to the mixture provided in (ii) or *vice versa*. Hydrochloric acid may be used as gas or preferably, in form of an aqueous solution. Most preferably, aqueous hydrochloric acid having a concentration in the range of from about 0.1 to 37 weight%, preferably from about 9 to 37 weight%, more preferably from about 18 to 37 weight% is used. Most preferably, concentrated hydrochloric acid having a concentration of about 37 weight% is used.

The molar ratio of omarigliptin and acid applied is in the range of from about 1.0 : 0.8 to 1.0 : 1.2, preferably from about 1.0 : 0.9 to 1.0 : 1.1 and most preferably the ratio is about 1.0 : 1.0.

In step (iv), the omarigliptin acid addition salt is separated from the reaction mixture. The separated omarigliptin acid addition salt may be in amorphous, pseudo-amorphous or crystalline form. Preferably, the separated omarigliptin acid addition salt is in crystalline form. Preferably, the separated omarigliptin acid addition salt is the non-hygroscopic acid addition salt of omarigliptin selected from the group consisting of omarigliptin hydrochloride, omarigliptin fumarate, omarigliptin *R*-mandelate, omarigliptin *S-*mandelate and omarigliptin *RS-*mandelate. More preferably, the separated omarigliptin acid addition salt is the crystalline non-hygroscopic acid addition salt of omarigliptin selected from the group consisting of crystalline omarigliptin hydrochloride, crystalline omarigliptin fumarate, crystalline omarigliptin *R-*mandelate, crystalline omarigliptin *S-*mandelate and crystalline omarigliptin *RS*-mandelate as described above.

Regarding separation in step (iv), no specific limitation exists provided that the non-hygroscopic acid addition salts of omarigliptin, preferably the crystalline non-hygroscopic acid addition salts of omarigliptin of the present invention can be separated from the reaction mixture.

Thus, separation can be carried out e.g. by partially evaporating the solvent, preferably by partially evaporating the solvent until precipitation or crystallization sets in or by completely evaporating the solvent. Separation can also be carried out e.g. by adding a suitable anti-solvent to the reaction mixture to initiate precipitation or crystallization of the non-hygroscopic acid addition salt or of the crystalline, non-hygroscopic acid addition salt of omarigliptin, respectively. Separation can also be carried out by combining partial solvent evaporation with the use of a suitable anti-solvent as described above.

Preferably, in step (iv), the non-hygroscopic, crystalline acid addition salt of omarigliptin is separated from the reaction mixture. Thus, step (iv) preferably further comprises
(iv.1) crystallizing the non-hygroscopic omarigliptin acid addition salt obtained in (iii) to obtain the non-hygroscopic crystalline acid addition salt of omarigliptin

Regarding crystallization in step (iv.1), no specific limitation exists provided that the non-hygroscopic crystalline acid addition salt of omarigliptin prepared in step (iii) is isolated. Thus, crystallization in step (iv.1) may be initiated by cooling the reaction mixture obtained in step (iii) to a temperature of about 40 °C or less, preferably of about 30 °C or less, more preferably of about 20 °C or less and most preferably of about 10 °C or less. In a preferred embodiment, the reaction mixture obtained in (iii) is first cooled to about room temperature and then further cooled to a temperature in the range of from about 2 to 8 °C. Seed crystals may be added to the reaction mixture in order to promote crystallization and to control crystal growth and particle size distribution of the crystalline product.

After separation of the non-hygroscopic acid addition salt of omarigliptin, preferably of the crystalline non-hygroscopic acid addition salt of omarigliptin from the reaction mixture, said addition salt or crystalline addition salt may optionally be collected. Thus, in a preferred embodiment, the process further comprises
(v) optionally collecting the non-hygroscopic acid addition salt of omarigliptin, preferably the crystalline non-hygroscopic acid addition salt of omarigliptin of step (iv).

Regarding collection, the non-hygroscopic acid addition salt of omarigliptin, preferably the crystalline non-hygroscopic acid addition salt of omarigliptin may be collected by any conventional method such as filtration or centrifugation, preferably by filtration.

Optionally, the non-hygroscopic acid addition salt of omarigliptin, preferably the crystalline non-hygroscopic acid addition salt of omarigliptin may be washed with a suitable solvent or solvent mixture comprising two or more suitable solvents. Thus, in a preferred embodiment, the process further comprises
(vi) optionally washing the non-hygroscopic acid addition salt of omarigliptin, preferably the crystalline non-hygroscopic acid addition salt of omarigliptin with a suitable solvent or solvent mixture.

Preferably, the suitable solvent comprises a water-miscible organic solvent and water, whereat the water-miscible organic solvent is selected from alcohols, ketones, nitriles, cyclic ethers or mixtures of two or more of them. More preferably, the solvent comprises an aqueous alcohol such as methanol, ethanol, 1-propanol, 2-propanol or mixtures of two or more thereof and most preferably the solvent is water.

Finally, the non-hygroscopic acid addition salt of omarigliptin, preferably the crystalline non-hygroscopic acid addition salt of omarigliptin may optionally be dried. Thus, in a preferred embodiment, the process further comprises
(vii) optionally drying the non-hygroscopic acid addition salt of omarigliptin, preferably the crystalline non-hygroscopic acid addition salt of omarigliptin.

Preferably, drying is carried out at a temperature of about 100 °C or less, preferably of about 80 °C or less, more preferably of about 60 °C or less and most preferably drying is carried out at a temperature of about 40 °C or less for example at about room temperature. Drying may be performed for a period in the range of from about 1 to 72 hours, preferably from about 2 to 48 hours, more preferably from about 4 to 24 hours and most preferably from about 6 to 18 hours. Drying may be performed under vacuum preferably at about 100 mbar or less, more preferably at about 50 mbar or less and most preferably at about 30 mbar or less, for example at about 20 mbar or less.

As can be seen from table A the exemplary salts of the present invention show a water uptake of not more than 2.0 weight%, based on the weight of the respective omarigliptin salt, when measured with GMS at a relative humidity in the range of from 0 to 95% and a temperature of 25.0 °C. The non-hygroscopic acid addition salts of the present invention were found to be chemically very stable even when subjected to severe moisture stress such as to atmospheres having a temperature of 40 °C and 75% relative humidity. As shown in table A below no significant increase in total impurities was observed after one week at accelerated stress conditions. In addition, no change in the crystal structure was observed after the GMS experiment as well as the stress test, which was confirmed by PXRD.

**Table A: Summary of results from GMS experiments and accelerated stress tests with omarigliptin salts of the present invention**

| Crystalline omarigliptin salt | mass change between 0-95% RH at 25.0°C ± 0.1 °C | change in total impurities after one week open storage at 40 °C/ 75% RH |
|---|---|---|
| hydrochloride | 2.0% | + 0.5 area% |
| fumarate | 1.4% | - 0.8 area% |
| *R*-mandelate | 0.7% | 0.0 area% |
| *S-*mandelate | 1.0% | - 0.4 area% |
| *RS*-mandelate | 0.8% | + 0.6 area% |

Hence, the omarigliptin acid addition salts of the present invention are especially suitable to be used in and for the preparation of a pharmaceutical composition.

Therefore, in a further aspect, the present invention relates to the use of a non-hygroscopic acid addition salt of omarigliptin, preferably of a crystalline non-hygroscopic acid addition salt of omarigliptin according to the present invention for the preparation of a pharmaceutical composition.

Furthermore, the present invention relates to a pharmaceutical composition comprising an effective amount of a non-hygroscopic acid addition salt of omarigliptin, preferably of a crystalline non-hygroscopic acid addition salt of omarigliptin according to the present invention and at least one pharmaceutically acceptable excipient.

For example, microcrystalline cellulose and mannitol can be used as diluent, croscarmellose sodium as a disintegrant and magnesium stearate as a lubricant in the pharmaceutical composition of the present invention.

Preferably, the pharmaceutical composition of the present invention is a tablet, most preferably a film-coated tablet.

Film coated tablets of the present invention may be prepared by blending a crystalline omarigliptin acid addition salt of the present invention with one or more pharmaceutical acceptable excipients, lubricating with magnesium stearate, followed by compression and subsequent film coating.

In addition, the present invention relates to a pharmaceutical composition comprising an effective amount of a non-hygroscopic acid addition salt of omarigliptin, preferably of a crystalline non-hygroscopic acid addition salt of omarigliptin according to the present invention and at least one pharmaceutically acceptable excipient for use as a medicament.

Finally, the present invention relates to a pharmaceutical composition comprising an effective amount of a non-hygroscopic acid addition salt of omarigliptin, preferably of a crystalline non-hygroscopic acid addition salt of omarigliptin according to the present invention and at least one pharmaceutically acceptable excipient for use in the treatment of a condition selected from diabetes type 2, obesity and high blood pressure.

### Embodiment section

Aspects, advantageous features and preferred embodiments of the present invention are summarized in the following items:
1) An acid addition salt of omarigliptin, wherein the omarigliptin acid addition salt shows a water uptake of at most 2.0 weight%, based on the weight of the omarigliptin acid addition salt, when measured with gravimetric moisture sorption at a relative humidity in the range of from 0 to 95% and a temperature of 25.0 ± 0.1 °C.
2) An acid addition salt of omarigliptin, wherein the omarigliptin acid addition salt shows a water uptake of at most 1.4 weight%, based on the weight of the omarigliptin acid addition salt, when measured with gravimetric moisture sorption at a relative humidity in the range of from 0 to 95% and a temperature of 25.0 ± 0.1 °C.
3) An acid addition salt of omarigliptin, wherein the omarigliptin acid addition salt shows a water uptake of at most 1.0 weight%, based on the weight of the omarigliptin acid addition salt, when measured with gravimetric moisture sorption at a relative humidity in the range of from 0 to 95% and a temperature of 25.0 ± 0.1 °C.
4) An acid addition salt of omarigliptin, wherein the omarigliptin acid addition salt shows a water uptake of at most 0.8 weight%, based on the weight of the omarigliptin acid addition salt, when measured with gravimetric moisture sorption at a relative humidity in the range of from 0 to 95% and a temperature of 25.0 ± 0.1 °C.
5) An acid addition salt of omarigliptin, wherein the omarigliptin acid addition salt shows a water uptake of at most 0.7 weight%, based on the weight of the omarigliptin acid addition salt, when measured with gravimetric moisture sorption at a relative humidity in the range of from 0 to 95% and a temperature of 25.0 ± 0.1 °C.
6) The omarigliptin acid addition salt according to any one of the preceding items, wherein the omarigliptin acid addition salt is crystalline.
7) The omarigliptin acid addition salt according to any one of the preceding items characterized by a molar ratio of omarigliptin and acid in the range of from 1.0: 0.8 to 1.2.
8) The omarigliptin acid addition salt according to any one of items 1 to 7 characterized by a molar ratio of omarigliptin and acid in the range of from 1.0:0.9 to 1.0:1.1.
9) The omarigliptin acid addition salt according to any one of items 1 to 7 characterized by a molar ratio of omarigliptin and acid of 1.0:1.0.
10) The omarigliptin acid addition salt according to any one of the preceding items characterized by showing a weight loss of 0.5 weight% or less, based on the weight of the omarigliptin acid addition salt, when measured with thermogravimetric analysis at a temperature in the range of from 25 to 200 °C and a heating rate of 10 K/min.
11) The omarigliptin acid addition salt according to any one of items 1 to 9 characterized by showing a weight loss of 0.4 weight% or less, based on the weight of the omarigliptin acid addition salt, when measured with thermogravimetric analysis at a temperature in the range of from 25 to 200 °C and a heating rate of 10 K/min.
12) The omarigliptin acid addition salt according to any one of items 1 to 9 characterized by showing a weight loss of 0.3 weight% or less, based on the weight of the omarigliptin acid addition salt, when measured with thermogravimetric analysis at a temperature in the range of from 25 to 200 °C and a heating rate of 10 K/min.
13) The omarigliptin acid addition salt according to any one of items 1 to 9 characterized by showing a weight loss of 0.2 weight% or less, based on the weight of the omarigliptin acid addition salt, when measured with thermogravimetric analysis at a temperature in the range of from 25 to 200 °C and a heating rate of 10 K/min.
14) The omarigliptin acid addition salt according to any one of items 1 to 9 characterized by showing a weight loss of 0.1 weight% or less, based on the weight of the omarigliptin acid addition salt, when measured with thermogravimetric analysis at a temperature in the range of from 25 to 200 °C and a heating rate of 10 K/min.
15) The omarigliptin acid addition salt according to any one of the preceding items, wherein the acid addition salt is non-solvated.
16) The omarigliptin acid addition salt according any one of the preceding items, wherein the omarigliptin acid addition salt is selected from the group of omarigliptin hydrochloride, omarigliptin fumarate, omarigliptin *R*-mandelate, omarigliptin *S-*mandelate and omarigliptin *RS-*mandelate.
17) The omarigliptin acid addition salt according to item 16, wherein the omarigliptin acid addition salt is crystalline omarigliptin hydrochloride.
18) Crystalline omarigliptin hydrochloride according to item 17 characterized by having a powder X-ray diffractogram comprising reflections at 2-Theta angles selected from the group of (18.7 ± 0.2)°, (21.0 ± 0.2)° and (24.2 ± 0.2)°, when measured with Cu-Kalpha_{1,2} radiation at a wavelength of 0.15419 nm and a temperature in the range of from 20 to 30 °C.
19) Crystalline omarigliptin hydrochloride according to item 17 characterized by having a powder X-ray diffractogram comprising reflections at 2-Theta angles selected from the group of (10.1 ± 0.2)°, (18.7 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.
20) Crystalline omarigliptin hydrochloride according to item 17 characterized by having a powder X-ray diffractogram comprising reflections at 2-Theta angles selected from the group of (8.7 ± 0.2)°, (10.1 ± 0.2)°, (18.7 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.
21) Crystalline omarigliptin hydrochloride according to item 17 characterized by having a powder X-ray diffractogram comprising reflections at 2-Theta angles selected from the group of (4.8 ± 0.2)°, (8.7 ± 0.2)°, (10.1 ± 0.2)°, (18.7 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.
22) Crystalline omarigliptin hydrochloride according to item 17 characterized by having a powder X-ray diffractogram comprising reflections at 2-Theta angles selected from the group of (4.8 ± 0.2)°, (8.7 ± 0.2)°, (10.1 ± 0.2)°, (16.5 ± 0.2)°, (18.7 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.
23) Crystalline omarigliptin hydrochloride according to item 17 characterized by having a powder X-ray diffractogram comprising reflections at 2-Theta angles selected from the group of (4.8 ± 0.2)°, (8.7 ± 0.2)°, (10.1 ± 0.2)°, (16.5 ± 0.2)°, (17.4 ± 0.2)°, (18.7 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.
24) Crystalline omarigliptin hydrochloride according to item 17 characterized by having a powder X-ray diffractogram comprising reflections at 2-Theta angles selected from the group of (4.8 ± 0.2)°, (8.7 ± 0.2)°, (10.1 ± 0.2)°, (16.5 ± 0.2)°, (17.4 ± 0.2)°, (18.7 ± 0.2)°, (20.3 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.
25) Crystalline omarigliptin hydrochloride according to item 17 characterized by having a powder X-ray diffractogram comprising reflections at 2-Theta angles selected from the group of (4.8 ± 0.2)°, (8.7 ± 0.2)°, (10.1 ± 0.2)°, (16.5 ± 0.2)°, (17.4 ± 0.2)°, (18.7 ± 0.2)°, (20.3 ± 0.2)°, (20.8 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.
26) Crystalline omarigliptin hydrochloride according to item 17 characterized by having a powder X-ray diffractogram essentially the same as displayed in figure 1 of the present invention, when measured with Cu-Kalpha₁,₂ radiation at a wavelength of 0.15419 nm and a temperature in the range of from 20 to 30 °C.
27) Crystalline omarigliptin hydrochloride according to any one of items 17 to 26 characterized by having a Fourier transform infrared spectrum comprising peaks at wavenumbers of (2849 ± 2) cm⁻¹, (2794 ± 2) cm⁻¹, (1515 ± 2) cm⁻¹, (1326 ± 2) cm⁻¹ and (1274 ± 2) cm⁻¹, when measured with a diamond ATR cell at a temperature in the range of from 20 to 30 °C.
28) Crystalline omarigliptin hydrochloride according to any one of items 17 to 26 characterized by having a Fourier transform infrared spectrum essentially the same as displayed in figure 2 of the present invention, when measured with a diamond ATR cell at a temperature in the range of from 20 to 30 °C.
29) Crystalline omarigliptin hydrochloride according to any one of items 17 to 28 characterized by having a differential scanning calorimetric curve showing no thermal event up to a temperature of 200 °C, when measured at a heating rate of 10 K/min.
30) Crystalline omarigliptin hydrochloride according to any one of items 17 to 29 characterized by exhibiting no melting point.
31) Crystalline omarigliptin hydrochloride according to any one of items 17 to 30 characterized by showing a weight loss of about 0.5 weight% or less, based on the weight of the crystalline omarigliptin hydrochloride, when measured with thermogravimetric analysis at a temperature in the range of from 25 to 200 °C and a heating rate of 10 K/min.
32) Crystalline omarigliptin hydrochloride according to any one of items 17 to 30 characterized by showing a weight loss of 0.4 weight% or less, based on the weight of the crystalline omarigliptin hydrochloride, when measured with thermogravimetric analysis at a temperature in the range of from 25 to 200 °C and a heating rate of 10 K/min.
33) Crystalline omarigliptin hydrochloride according to any one of items 17 to 30 characterized by showing a weight loss of 0.3 weight% or less, based on the weight of the crystalline omarigliptin hydrochloride, when measured with thermogravimetric analysis at a temperature in the range of from 25 to 200 °C and a heating rate of 10 K/min.
34) Crystalline omarigliptin hydrochloride according to any one of items 17 to 30 characterized by showing a weight loss of 0.2 weight% or less, based on the weight of the crystalline omarigliptin hydrochloride, when measured with thermogravimetric analysis at a temperature in the range of from 25 to 200 °C and a heating rate of 10 K/min.
35) Crystalline omarigliptin hydrochloride according to any one of items 17 to 30 characterized by showing a weight loss of 0.1 weight% or less, based on the weight of the crystalline omarigliptin hydrochloride, when measured with thermogravimetric analysis at a temperature in the range of from 25 to 200 °C and a heating rate of 10 K/min.
36) Crystalline omarigliptin hydrochloride according to any one of items 17 to 35, wherein the crystalline omarigliptin hydrochloride is non-solvated.
37) Crystalline omarigliptin hydrochloride according to any one of items 17 to 36 characterized by showing a water uptake of at most 2.0 weight%, based on the weight of the crystalline omarigliptin hydrochloride, when measured with gravimetric moisture sorption from about 0 to 95% relative humidity at a temperature of 25.0 ± 0.1 °C.
38) Crystalline omarigliptin hydrochloride according to any one of items 17 to 37, wherein the crystalline omarigliptin hydrochloride is non-hygroscopic.
39) Crystalline omarigliptin hydrochloride according to any one of items 17 to 38, characterized by having a molar ratio of omarigliptin and hydrochloric acid in the range of from 1.0: 0.8 to 1.0:1.2.
40) Crystalline omarigliptin hydrochloride according to any one of items 17 to 38, characterized by having a molar ratio of omarigliptin and hydrochloric acid in the range of from 1.0: 0.9 to 1.0:1.1.
41) Crystalline omarigliptin hydrochloride according to any one of items 17 to 38, characterized by having a molar ratio of omarigliptin and hydrochloric acid of 1.0: 1.0.
42) The omarigliptin acid addition salt according to item 17, wherein the omarigliptin acid addition salt is crystalline omarigliptin fumarate.
43) Crystalline omarigliptin fumarate according to item 42, characterized by having a powder X-ray diffractogram comprising reflections at 2-Theta angles selected from the group of (13.8 ± 0.2)°, (19.7 ± 0.2)° and (21.2 ± 0.2)°, when measured with Cu-Kalpha₁,₂ radiation at a wavelength of 0.15419 nm and a temperature in the range of from 20 to 30 °C.
44) Crystalline omarigliptin fumarate according to item 42, characterized by having a powder X-ray diffractogram comprising reflections at 2-Theta angles selected from the group of (11.7 ± 0.2)°, (13.8 ± 0.2)°, (19.7 ± 0.2)° and (21.2 ± 0.2)°, when measured with Cu-Kalpha₁,₂ radiation at a wavelength of 0.15419 nm and a temperature in the range of from 20 to 30 °C.
45) Crystalline omarigliptin fumarate according to item 42, characterized by having a powder X-ray diffractogram comprising reflections at 2-Theta angles selected from the group of (6.9 ± 0.2)°, (11.7 ± 0.2)°, (13.8 ± 0.2)°, (19.7 ± 0.2)° and (21.2 ± 0.2)°, when measured with Cu-Kalpha₁,₂ radiation at a wavelength of 0.15419 nm and a temperature in the range of from 20 to 30 °C.
46) Crystalline omarigliptin fumarate according to item 42, characterized by having a powder X-ray diffractogram comprising reflections at 2-Theta angles selected from the group of (6.9 ± 0.2)°, (8.9 ± 0.2)°, (11.7 ± 0.2)°, (13.8 ± 0.2)°, (19.7 ± 0.2)° and (21.2 ± 0.2)°, when measured with Cu-Kalpha₁,₂ radiation at a wavelength of 0.15419 nm and a temperature in the range of from 20 to 30 °C.
47) Crystalline omarigliptin fumarate according to item 42, characterized by having a powder X-ray diffractogram comprising reflections at 2-Theta angles selected from the group of (6.9 ± 0.2)°, (8.9 ± 0.2)°, (10.8 ± 0.2)°, (11.7 ± 0.2)°, (13.8 ± 0.2)°, (19.7 ± 0.2)° and (21.2 ± 0.2)°, when measured with Cu-Kalpha₁,₂ radiation at a wavelength of 0.15419 nm and a temperature in the range of from 20 to 30 °C.
48) Crystalline omarigliptin fumarate according to item 42, characterized by having a powder X-ray diffractogram comprising reflections at 2-Theta angles selected from the group of (6.9 ± 0.2)°, (8.9 ± 0.2)°, (10.8 ± 0.2)°, (11.7 ± 0.2)°, (13.8 ± 0.2)°, (19.7 ± 0.2)°, (21.2 ± 0.2)° and (22.1 ± 0.2)°, when measured with Cu-Kalpha₁,₂ radiation at a wavelength of 0.15419 nm and a temperature in the range of from 20 to 30 °C.
49) Crystalline omarigliptin fumarate according to item 42, characterized by having a powder X-ray diffractogram comprising reflections at 2-Theta angles selected from the group of (6.9 ± 0.2)°, (8.9 ± 0.2)°, (10.8 ± 0.2)°, (11.7 ± 0.2)°, (13.8 ± 0.2)°, (19.7 ± 0.2)°, (21.2 ± 0.2)°, (22.1 ± 0.2)° and (22.5 ± 0.2)°, when measured with Cu-Kalpha₁,₂ radiation at a wavelength of 0.15419 nm and a temperature in the range of from 20 to 30 °C.
50) Crystalline omarigliptin fumarate according to item 42, characterized by having a powder X-ray diffractogram comprising reflections at 2-Theta angles selected from the group of (6.9 ± 0.2)°, (8.9 ± 0.2)°, (10.8 ± 0.2)°, (11.7 ± 0.2)°, (13.8 ± 0.2)°, (19.7 ± 0.2)°, (21.2 ± 0.2)°, (22.1 ± 0.2)°, (22.5 ± 0.2)° and (24.8 ± 0.2)°, when measured with Cu-Kalpha₁,₂ radiation at a wavelength of 0.15419 nm and a temperature in the range of from 20 to 30 °C.
51) Crystalline omarigliptin fumarate according to item 42, characterized by having a powder X-ray diffractogram essentially the same as displayed in figure 6 of the present invention, when measured with Cu-Kalpha₁,₂ radiation at a wavelength of 0.15419 nm and a temperature in the range of from 20 to 30 °C.
52) Crystalline omarigliptin fumarate according to any one of items 42 to 51, characterized by having a Fourier transform infrared spectrum comprising peaks at wavenumbers of (3301 ± 2) cm⁻¹, (3088 ± 2) cm⁻¹, (1573 ± 2) cm⁻¹, (1359 ± 2) cm⁻¹ and (1165 ± 2) cm⁻¹, when measured with a diamond ATR cell at a temperature in the range of from 20 to 30 °C.
53) Crystalline omarigliptin fumarate according to any one of items 42 to 51, characterized by having a Fourier transform infrared spectrum essentially the same as displayed in figure 6 of the present invention, when measured with a diamond ATR cell at a temperature in the range of from 20 to 30 °C.
54) Crystalline omarigliptin fumarate according to any one of items 42 to 53, characterized by having a differential scanning calorimetric curve showing no thermal event up to a temperature of 170 °C, when measured at a heating rate of 10 K/min.
55) Crystalline omarigliptin fumarate according to any one of items 42 to 53, characterized by having a melting point at a temperature in the range of from 175 to 201 °C, when measured with differential scanning calorimetry at a heating rate of 10 K/min.
56) Crystalline omarigliptin fumarate according to any one of items 42 to 55, characterized by showing a weight loss of 0.5 weight% or less, based on the weight of the crystalline omarigliptin fumarate, when measured with thermogravimetric analysis at a temperature in the range of from 25 to 200 °C and a heating rate of 10 K/min.
57) Crystalline omarigliptin fumarate according to any one of items 42 to 55, characterized by showing a weight loss of 0.4 weight% or less, based on the weight of the crystalline omarigliptin fumarate, when measured with thermogravimetric analysis at a temperature in the range of from 25 to 200 °C and a heating rate of 10 K/min.
58) Crystalline omarigliptin fumarate according to any one of items 42 to 55, characterized by showing a weight loss of 0.3 weight% or less, based on the weight of the crystalline omarigliptin fumarate, when measured with thermogravimetric analysis at a temperature in the range of from 25 to 200 °C and a heating rate of 10 K/min.
59) Crystalline omarigliptin fumarate according to any one of items 42 to 55, characterized by showing a weight loss of 0.2 weight% or less, based on the weight of the crystalline omarigliptin fumarate, when measured with thermogravimetric analysis at a temperature in the range of from 25 to 200 °C and a heating rate of 10 K/min.
60) Crystalline omarigliptin fumarate according to any one of items 42 to 55, characterized by showing a weight loss of 0.1 weight% or less, based on the weight of the crystalline omarigliptin fumarate, when measured with thermogravimetric analysis at a temperature in the range of from 25 to 200 °C and a heating rate of 10 K/min.
61) Crystalline omarigliptin fumarate according to any one of items 42 to 60, wherein the crystalline omarigliptin fumarate is non-solvated.
62) Crystalline omarigliptin fumarate according to any one of items 42 to 61, characterized by showing a water uptake of at most 2.0 weight%, based on the weight of the crystalline omarigliptin fumarate, when measured with gravimetric moisture sorption from about 0 to 95% relative humidity at a temperature of 25.0 ± 0.1 °C.
63) Crystalline omarigliptin fumarate according to any one of items 42 to 61, characterized by showing a water uptake of at most 1.5 weight%, based on the weight of the crystalline omarigliptin fumarate, when measured with gravimetric moisture sorption from about 0 to 95% relative humidity at a temperature of 25.0 ± 0.1 °C.
64) Crystalline omarigliptin fumarate according to any one of items 42 to 61, wherein the crystalline omarigliptin fumarate is non-hygroscopic.
65) Crystalline omarigliptin fumarate according to any one of items 42 to 64, characterized by having a molar ratio of omarigliptin and fumaric acid in the range of from 1.0: 0.8 to 1.0:1.2.
66) Crystalline omarigliptin fumarate according to any one of items 42 to 64, characterized by having a molar ratio of omarigliptin and fumaric acid in the range of from 1.0: 0.9 to 1.0:1.1.
67) Crystalline omarigliptin fumarate according to any one of items 42 to 64, characterized by characterized by having a molar ratio of omarigliptin and fumaric acid of 1.0:1.0.
68) The omarigliptin acid addition salt according to item 17, wherein the omarigliptin acid addition salt is crystalline omarigliptin *R*-mandelate.
69) Crystalline omarigliptin *R*-mandelate according to item 68, characterized by having a powder X-ray diffractogram comprising reflections at 2-Theta angles selected from the group of (6.3 ± 0.2)°, (17.9 ± 0.2)° and (21.9 ± 0.2)°, when measured with Cu-Kalpha_{1,2} radiation at a wavelength of 0.15419 nm and a temperature in the range of from 20 to 30 °C.
70) Crystalline omarigliptin *R*-mandelate according to item 68, characterized by having a powder X-ray diffractogram comprising reflections at 2-Theta angles selected from the group of (6.3 ± 0.2)°, (17.9 ± 0.2)°, (21.9 ± 0.2)° and (28.0 ± 0.2)°, when measured with Cu-Kalpha₁,₂ radiation at a wavelength of 0.15419 nm and a temperature in the range of from 20 to 30 °C.
71) Crystalline omarigliptin *R*-mandelate according to item 68, characterized by having a powder X-ray diffractogram comprising reflections at 2-Theta angles selected from the group of (6.3 ± 0.2)°, (12.6± 0.2)°, (17.9 ± 0.2)°, (21.9 ± 0.2)° and (28.0 ± 0.2)°, when measured with Cu-Kalpha_{1,2} radiation at a wavelength of 0.15419 nm and a temperature in the range of from 20 to 30 °C.
72) Crystalline omarigliptin *R*-mandelate according to item 68, characterized by having a powder X-ray diffractogram comprising reflections at 2-Theta angles selected from the group of (6.3 ± 0.2)°, (7.1 ± 0.2)°, (12.6 ± 0.2)°, (17.9 ± 0.2)°, (21.9 ± 0.2)° and (28.0 ± 0.2)°, when measured with Cu-Kalpha₁,₂ radiation at a wavelength of 0.15419 nm and a temperature in the range of from 20 to 30 °C.
73) Crystalline omarigliptin *R*-mandelate according to item 68, characterized by having a powder X-ray diffractogram comprising reflections at 2-Theta angles selected from the group of (6.3 ± 0.2)°, (7.1 ± 0.2)°, (12.6 ± 0.2)°, (15.1 ± 0.2)°, (17.9 ± 0.2)°, (21.9 ± 0.2)° and (28.0 ± 0.2)°, when measured with Cu-Kalpha₁,₂ radiation at a wavelength of 0.15419 nm and a temperature in the range of from 20 to 30 °C.
74) Crystalline omarigliptin *R*-mandelate according to item 68, characterized by having a powder X-ray diffractogram comprising reflections at 2-Theta angles selected from the group of (6.3 ± 0.2)°, (7.1 ± 0.2)°, (12.6 ± 0.2)°, (15.1 ± 0.2)°, (15.3 ± 0.2)°, (17.9 ± 0.2)°, (21.9 ± 0.2)° and (28.0 ± 0.2)°, when measured with Cu-Kalpha₁,₂ radiation at a wavelength of 0.15419 nm and a temperature in the range of from 20 to 30 °C.
75) Crystalline omarigliptin *R*-mandelate according to item 68, characterized by having a powder X-ray diffractogram comprising reflections at 2-Theta angles selected from the group of (6.3 ± 0.2)°, (7.1 ± 0.2)°, (12.6 ± 0.2)°, (15.1 ± 0.2)°, (15.3 ± 0.2)°, (17.9 ± 0.2)°, (20.8 ± 0.2)°, (21.9 ± 0.2)° and (28.0 ± 0.2)°, when measured with Cu-Kalpha₁,₂ radiation at a wavelength of 0.15419 nm and a temperature in the range of from 20 to 30 °C.
76) Crystalline omarigliptin *R*-mandelate according to item 68, characterized by having a powder X-ray diffractogram comprising reflections at 2-Theta angles selected from the group of (6.3± 0.2)°, (7.1 ± 0.2)°, (12.6 ± 0.2)°, (15.1 ± 0.2)°, (15.3 ± 0.2)°, (17.9 ± 0.2)°, (20.8 ± 0.2)°, (21.9 ± 0.2)°, (23.2 ± 0.2)° and (28.0 ± 0.2)°, when measured with Cu-Kalpha₁,₂ radiation at a wavelength of 0.15419 nm and a temperature in the range of from 20 to 30 °C.
77) Crystalline omarigliptin *R*-mandelate according to item 68, characterized by having a powder X-ray diffractogram essentially the same as displayed in figure 9 of the present invention, when measured with Cu-Kalpha₁,₂ radiation at a wavelength of 0.15419 nm and a temperature in the range of from 20 to 30 °C.
78) Crystalline omarigliptin *R*-mandelate according to any one of items 68 to 77, characterized by having a Fourier transform infrared spectrum comprising peaks at wavenumbers of (3390 ± 2) cm⁻¹, (1636 ± 2) cm⁻¹, (1487 ± 2) cm⁻¹, (1371 ± 2) cm⁻¹ and (1037 ± 2) cm⁻¹, when measured with a diamond ATR cell at a temperature in the range of from 20 to 30 °C.
79) Crystalline omarigliptin *R*-mandelate according to any one of items 68 to 77, characterized by having a Fourier transform infrared spectrum essentially the same as displayed in figure 10 of the present invention, when measured with a diamond ATR cell at a temperature in the range of from 20 to 30 °C.
80) Crystalline omarigliptin *R*-mandelate according to any one of items 68 to 79, characterized by having a differential scanning calorimetric curve showing no thermal event up to a temperature of 200 °C, when measured at a heating rate of 10 K/min.
81) Crystalline omarigliptin *R*-mandelate according to any one of items 68 to 80, characterized by having a melting point at a temperature in the range of from 204 to 206 °C, when measured with differential scanning calorimetry at a heating rate of 10 K/min.
82) Crystalline omarigliptin *R*-mandelate according to any one of items 68 to 81, characterized by showing a weight loss of about 0.5 weight% or less, based on the weight of the crystalline omarigliptin *R*-mandelate, when measured with thermogravimetric analysis at a temperature in the range of from 25 to 200 °C and a heating rate of 10 K/min.
83) Crystalline omarigliptin *R*-mandelate according to any one of items 68 to 81, characterized by showing a weight loss of 0.4 weight% or less, based on the weight of the crystalline omarigliptin *R*-mandelate, when measured with thermogravimetric analysis at a temperature in the range of from 25 to 200 °C and a heating rate of 10 K/min.
84) Crystalline omarigliptin *R*-mandelate according to any one of items 68 to 81, characterized by showing a weight loss of 0.3 weight% or less, based on the weight of the crystalline omarigliptin *R*-mandelate, when measured with thermogravimetric analysis at a temperature in the range of from 25 to 200 °C and a heating rate of 10 K/min.
85) Crystalline omarigliptin *R*-mandelate according to any one of items 68 to 81, characterized by showing a weight loss of 0.2 weight% or less, based on the weight of the crystalline omarigliptin *R*-mandelate, when measured with thermogravimetric analysis at a temperature in the range of from 25 to 200 °C and a heating rate of 10 K/min.
86) Crystalline omarigliptin *R*-mandelate according to any one of items 68 to 81, characterized by showing a weight loss of 0.1 weight% or less, based on the weight of the crystalline omarigliptin *R*-mandelate, when measured with thermogravimetric analysis at a temperature in the range of from 25 to 200 °C and a heating rate of 10 K/min.
87) Crystalline omarigliptin *R*-mandelate according to any one of items 68 to 86, wherein the crystalline omarigliptin *R*-mandelate is non-solvated.
88) Crystalline omarigliptin *R*-mandelate according to anyone of items 68 to 87, characterized by showing a water uptake of at most 2.0 weight%, based on the weight of the crystalline omarigliptin *R*-mandelate, when measured with gravimetric moisture sorption from about 0 to 95% relative humidity at a temperature of 25.0 ± 0.1 °C.
89) Crystalline omarigliptin *R*-mandelate according to anyone of items 68 to 87, characterized by showing a water uptake of at most 1.5 weight%, based on the weight of the crystalline omarigliptin *R*-mandelate, when measured with gravimetric moisture sorption from about 0 to 95% relative humidity at a temperature of 25.0 ± 0.1 °C.
90) Crystalline omarigliptin *R*-mandelate according to anyone of items 68 to 87, characterized by showing a water uptake of at most 1.0 weight%, based on the weight of the crystalline omarigliptin *R*-mandelate, when measured with gravimetric moisture sorption from about 0 to 95% relative humidity at a temperature of 25.0 ± 0.1 °C.
91) Crystalline omarigliptin *R*-mandelate according to anyone of items 68 to 87, characterized by showing a water uptake of at most 0.8 weight%, based on the weight of the crystalline omarigliptin *R*-mandelate, when measured with gravimetric moisture sorption from about 0 to 95% relative humidity at a temperature of 25.0 ± 0.1 °C.
92) Crystalline omarigliptin *R*-mandelate according to anyone of items 68 to 91, wherein the crystalline omarigliptin *R*-mandelate is non-hygroscopic.
93) Crystalline omarigliptin *R*-mandelate according to an yone of items 68 to 92, characterized by having a molar ratio of omarigliptin and *R*-mandelic acid in the range of from 1.0: 0.8 to 1.0:1.2.
94) Crystalline omarigliptin *R*-mandelate according to anyone of items 68 to 92, characterized by having a molar ratio of omarigliptin and *R*-mandelic acid in the range of from 1.0: 0.9 to 1.0:1.1.
95) Crystalline omarigliptin *R*-mandelate according to anyone of items 68 to 92, characterized by having a molar ratio of omarigliptin and *R*-mandelic acid of 1.0: 1.0.
96) The omarigliptin acid addition salt according to item 17, wherein the omarigliptin acid addition salt is crystalline omarigliptin *S*-mandelate.
97) The crystalline omarigliptin *S-*mandelate according to item 96, characterized by having a powder X-ray diffractogram comprising reflections at 2-Theta angles selected from the group of (16.3 ± 0.2)°, (18.6 ± 0.2)° and (20.7 ± 0.2)°, when measured with Cu-Kalpha_{1,2} radiation at a wavelength of 0.15419 nm and a temperature in the range of from 20 to 30 °C.
98) The crystalline omarigliptin *S-*mandelate according to item 96, characterized by having a powder X-ray diffractogram comprising reflections at 2-Theta angles selected from the group of (7.1 ± 0.2)°, (16.3 ± 0.2)°, (18.6 ± 0.2)° and (20.7 ± 0.2)°, when measured with Cu-Kalpha₁,₂ radiation at a wavelength of 0.15419 nm and a temperature in the range of from 20 to 30 °C.
99) The crystalline omarigliptin *S-*mandelate according to item 96, characterized by having a powder X-ray diffractogram comprising reflections at 2-Theta angles selected from the group of (7.1 ± 0.2)°, (8.8± 0.2)°, (16.3 ± 0.2)°, (18.6 ± 0.2)° and (20.7 ± 0.2)°, when measured with Cu-Kalpha_{1,2} radiation at a wavelength of 0.15419 nm and a temperature in the range of from 20 to 30 °C.
100) The crystalline omarigliptin *S-*mandelate according to item 96, characterized by having a powder X-ray diffractogram comprising reflections at 2-Theta angles selected from the group of (7.1 ± 0.2)°, (8.8 ± 0.2)°, (16.3 ± 0.2)°, (18.2 ± 0.2)°, (18.6 ± 0.2)° and (20.7 ± 0.2)°, when measured with Cu-Kalpha_{1,2} radiation at a wavelength of 0.15419 nm and a temperature in the range of from 20 to 30 °C.
101) The crystalline omarigliptin S-mandelate according to item 96, characterized by having a powder X-ray diffractogram comprising reflections at 2-Theta angles selected from the group of (7.1 ± 0.2)°, (8.8 ± 0.2)°, (16.3 ± 0.2)°, (17.4 ± 0.2)°, (18.2 ± 0.2)°, (18.6 ± 0.2)° and (20.7± 0.2)°, when measured with Cu-Kalpha_{1,2} radiation at a wavelength of 0.15419 nm and a temperature in the range of from 20 to 30 °C.
102) The crystalline omarigliptin S-mandelate according to item 96, characterized by having a powder X-ray diffractogram comprising reflections at 2-Theta angles selected from the group of (7.1 ± 0.2)°, (8.8 ± 0.2)°, (16.3 ± 0.2)°, (17.4 ± 0.2)°, (18.2 ± 0.2)°, (18.6 ± 0.2)°, (20.7 ± 0.2)° and (21.0 ± 0.2)°, when measured with Cu-Kalpha_{1,2} radiation at a wavelength of 0.15419 nm and a temperature in the range of from 20 to 30 °C.
103) The crystalline omarigliptin S-mandelate according to item 96, characterized by having a powder X-ray diffractogram comprising reflections at 2-Theta angles selected from the group of (7.1 ± 0.2)°, (8.8 ± 0.2)°, (16.3 ± 0.2)°, (17.4 ± 0.2)°, (18.2 ± 0.2)°, (18.6 ± 0.2)°, (20.7 ± 0.2)°, (21.0 ± 0.2)° and (22.8 ± 0.2)°, when measured with Cu-Kalpha_{1,2} radiation at a wavelength of 0.15419 nm and a temperature in the range of from 20 to 30 °C.
104) The crystalline omarigliptin S-mandelate according to item 96, characterized by having a powder X-ray diffractogram comprising reflections at 2-Theta angles selected from the group of (7.1± 0.2)°, (8.8 ± 0.2)°, (14.9 ± 0.2)°, (16.3 ± 0.2)°, (17.4 ± 0.2)°, (18.2 ± 0.2)°, (18.6 ± 0.2)°, (20.7 ± 0.2)°, (21.0 ± 0.2)° and (22.8 ± 0.2)°, when measured with Cu-Kalpha_{1,2} radiation at a wavelength of 0.15419 nm and a temperature in the range of from 20 to 30 °C.
105) The crystalline omarigliptin S-mandelate according to item 96, characterized by having a powder X-ray diffractogram essentially the same as displayed in figure 13 of the present invention, when measured with Cu-Kalpha_{1,2} radiation at a wavelength of 0.15419 nm and a temperature in the range of from 20 to 30 °C.
106) The crystalline omarigliptin S-mandelate according to any one of items 96 to 105, characterized by having a Fourier transform infrared spectrum comprising peaks at wavenumbers of (3292 ± 2) cm⁻¹, (1544 ± 2) cm⁻¹, (1432 ± 2) cm⁻¹, (1365 ± 2) cm⁻¹ and (1218 ± 2) cm⁻¹, when measured with a diamond ATR cell at a temperature in the range of from 20 to 30 °C.
107) The crystalline omarigliptin S-mandelate according to any one of items 96 to 105, characterized by having a Fourier transform infrared spectrum essentially the same as displayed in figure 14 of the present invention, when measured with a diamond ATR cell at a temperature in the range of from 20 to 30 °C.
108) The crystalline omarigliptin S-mandelate according to any one of items 96 to 107, characterized by having a differential scanning calorimetric curve showing no thermal event up to a temperature of 180 °C, when measured at a heating rate of 10 K/min.
109) The crystalline omarigliptin S-mandelate according to any one of items 96 to 108, characterized by having a melting point at a temperature in the range of from 185 to 187 °C, when measured with differential scanning calorimetry at a heating rate of 10 K/min.
110) The crystalline omarigliptin *S*-mandelate according to any one of items 96 to 109, characterized by showing a weight loss of 0.5 weight% or less, based on the weight of the crystalline omarigliptin *S*-mandelate, when measured with thermogravimetric analysis at a temperature in the range of from 25 to 200 °C and a heating rate of 10 K/min.
111) The crystalline omarigliptin S-mandelate according to any one of items 96 to 109, characterized by showing a weight loss of 0.4 weight% or less, based on the weight of the crystalline omarigliptin S-mandelate, when measured with thermogravimetric analysis at a temperature in the range of from 25 to 200 °C and a heating rate of 10 K/min.
112) The crystalline omarigliptin S-mandelate according to any one of items 96 to 109, characterized by showing a weight loss of 0.3 weight% or less, based on the weight of the crystalline omarigliptin *S*-mandelate, when measured with thermogravimetric analysis at a temperature in the range of from 25 to 200 °C and a heating rate of 10 K/min.
113) The crystalline omarigliptin *S*-mandelate according to any one of items 96 to 109, characterized by showing a weight loss of 0.2 weight% or less, based on the weight of the crystalline omarigliptin *S*-mandelate, when measured with thermogravimetric analysis at a temperature in the range of from 25 to 200 °C and a heating rate of 10 K/min.
114) The crystalline omarigliptin *S*-mandelate according to any one of items 96 to 109, characterized by showing a weight loss of 0.1 weight% or less, based on the weight of the crystalline omarigliptin *S*-mandelate, when measured with thermogravimetric analysis at a temperature in the range of from 25 to 200 °C and a heating rate of 10 K/min.
115) The crystalline omarigliptin *S*-mandelate according to any one of items 96 to 114, wherein the crystalline omarigliptin *S*-mandelate is non-solvated.
116) Crystalline omarigliptin *S*-mandelate according to anyone of items 96 to 115 characterized by showing a water uptake of at most 2.0 weight%, based on the weight of the crystalline omarigliptin *S*-mandelate, when measured with gravimetric moisture sorption from about 0 to 95% relative humidity at a temperature of 25.0 ± 0.1 °C.
117) The crystalline omarigliptin *S*-mandelate according to any one of items 96 to 116, characterized by showing a water uptake of at most 1.5 weight%, based on the weight of the crystalline omarigliptin *S*-mandelate, when measured with gravimetric moisture sorption from about 0 to 95% relative humidity at a temperature of 25.0 ± 0.1 °C.
118) The crystalline omarigliptin *S*-mandelate according to any one of items 96 to 115, characterized by showing a water uptake of at most 1.0 weight%, based on the weight of the crystalline omarigliptin *S*-mandelate, when measured with gravimetric moisture sorption from about 0 to 95% relative humidity at a temperature of 25.0 ± 0.1 °C.
119) The crystalline omarigliptin *S*-mandelate according to any one of items 96 to 118, wherein the crystalline omarigliptin *S*-mandelate is non-hygroscopic.
120) The crystalline omarigliptin *S*-mandelate according to any one of items 96 to 119, characterized by having a molar ratio of omarigliptin and *S*-mandelic acid in the range of from 1.0: 0.8 to 1.0:1.2.
121) The crystalline omarigliptin S-mandelate according to any one of items 96 to 119, characterized by having a molar ratio of omarigliptin and S-mandelic acid in the range of from 1.0: 0.9 to 1.0:1.1.
122) The crystalline omarigliptin *S*-mandelate according to any one of items 96 to 119, characterized by having a molar ratio of omarigliptin and *S*-mandelic acid of 1.0: 1.0.
123) The omarigliptin acid addition salt according to item 17, wherein the omarigliptin acid addition salt is crystalline omarigliptin *RS*-mandelate.
124) The crystalline omarigliptin *RS*-mandelate according to item 123, characterized by having a powder X-ray diffractogram comprising reflections at 2-Theta angles selected from the group of (6.3 ± 0.2)°, (12.7 ± 0.2)° and (18.6 ± 0.2)°, when measured with Cu-Kalpha_{1,2} radiation at a wavelength of 0.15419 nm and a temperature in the range of from 20 to 30 °C.
125) The crystalline omarigliptin *RS*-mandelate according to item 123, characterized by having a powder X-ray diffractogram comprising reflections at 2-Theta angles selected from the group of (6.3 ± 0.2)°, (7.1 ± 0.2)°, (12.7 ± 0.2)° and (18.6 ± 0.2)°, when measured with Cu-Kalpha₁,₂ radiation at a wavelength of 0.15419 nm and a temperature in the range of from 20 to 30 °C.
126) The crystalline omarigliptin *RS*-mandelate according to item 123, characterized by having a powder X-ray diffractogram comprising reflections at 2-Theta angles selected from the group of (6.3 ± 0.2)°, (7.1± 0.2)°, (8.8 ± 0.2)°, (12.7 ± 0.2)° and (18.6 ± 0.2)°, when measured with Cu-Kalpha_{1,2} radiation at a wavelength of 0.15419 nm and a temperature in the range of from 20 to 30 °C.
127) The crystalline omarigliptin *RS*-mandelate according to item 123, characterized by having a powder X-ray diffractogram comprising reflections at 2-Theta angles selected from the group of (6.3 ± 0.2)°, (7.1 ± 0.2)°, (8.8 ± 0.2)°, (12.7 ± 0.2)°, (16.3 ± 0.2)° and (18.6 ± 0.2)°, when measured with Cu-Kalpha_{1,2} radiation at a wavelength of 0.15419 nm and a temperature in the range of from 20 to 30 °C.
128) The crystalline omarigliptin *RS*-mandelate according to item 123, characterized by having a powder X-ray diffractogram comprising reflections at 2-Theta angles selected from the group of (6.3 ± 0.2)°, (7.1 ± 0.2)°, (8.8 ± 0.2)°, (12.7 ± 0.2)°, (16.3 ± 0.2)°, (18.6 ± 0.2)° and (20.7± 0.2)°, when measured with Cu-Kalpha_{1,2} radiation at a wavelength of 0.15419 nm and a temperature in the range of from 20 to 30 °C.
129) The crystalline omarigliptin *RS*-mandelate according to item 123, characterized by having a powder X-ray diffractogram comprising reflections at 2-Theta angles selected from the group of (6.3 ± 0.2)°, (7.1 ± 0.2)°, (8.8 ± 0.2)°, (12.7 ± 0.2)°, (16.3 ± 0.2)°, (18.6 ± 0.2)°, (20.7 ± 0.2)° and (21.7 ± 0.2)°, when measured with Cu-Kalpha_{1,2} radiation at a wavelength of 0.15419 nm and a temperature in the range of from 20 to 30 °C.
130) The crystalline omarigliptin *RS*-mandelate according to item 123, characterized by having a powder X-ray diffractogram comprising reflections at 2-Theta angles selected from the group of (6.3 ± 0.2)°, (7.1 ± 0.2)°, (8.8 ± 0.2)°, (12.7 ± 0.2)°, (16.3 ± 0.2)°, (18.6 ± 0.2)°, (20.7 ± 0.2)°, (21.7 ± 0.2)° and (23.3 ± 0.2)°, when measured with Cu-Kalpha_{1,2} radiation at a wavelength of 0.15419 nm and a temperature in the range of from 20 to 30 °C.
131) The crystalline omarigliptin *RS*-mandelate according to item 123, characterized by having a powder X-ray diffractogram comprising reflections at 2-Theta angles selected from the group of (6.3± 0.2)°, (7.1 ± 0.2)°, (8.8 ± 0.2)°, (12.7 ± 0.2)°, (15.4 ± 0.2)°, (16.3 ± 0.2)°, (18.6 ± 0.2)°, (20.7 ± 0.2)°, (21.7 ± 0.2)° and (23.3 ± 0.2)°, when measured with Cu-Kalpha_{1,2} radiation at a wavelength of 0.15419 nm and a temperature in the range of from 20 to 30 °C.
132) The crystalline omarigliptin *RS*-mandelate according to item 123, characterized by having a powder X-ray diffractogram essentially the same as displayed in figure 17 of the present invention, when measured with Cu-Kalpha_{1,2} radiation at a wavelength of 0.15419 nm and a temperature in the range of from 20 to 30 °C.
133) The crystalline omarigliptin *RS*-mandelate according to any one of the items 123 to 132, characterized by having a Fourier transform infrared spectrum comprising peaks at wavenumbers of (1635 ± 2) cm⁻¹, (1554 ± 2) cm⁻¹, (1367 ± 2) cm-¹, (1108 ± 2) cm⁻¹ and (926 ± 2) cm⁻¹, when measured with a diamond ATR cell at a temperature in the range of from 20 to 30 °C.
134) The crystalline omarigliptin *RS*-mandelate according to any one of the items 123 to 132, characterized by having a Fourier transform infrared spectrum essentially the same as displayed in figure 18 of the present invention, when measured with a diamond ATR cell at a temperature in the range of from 20 to 30 °C.
135) The crystalline omarigliptin *RS*-mandelate according to any one of the items 123 to 134, characterized by having a differential scanning calorimetric curve showing no thermal event up to a temperature of 175 °C, when measured at a heating rate of 10 K/min.
136) The crystalline omarigliptin *RS*-mandelate according to any one of the items 123 to 135, characterized by exhibiting a first melting point at a temperature in the range of from 180 to 183 °C and a second melting point at a temperature in the range of from 190 to 198 °C, when measured with differential scanning calorimetry at a heating rate of 10 K/min.
137) The crystalline omarigliptin *RS*-mandelate according to any one of the items 123 to 136, characterized by showing a weight loss of 0.5 weight% or less, based on the weight of the crystalline omarigliptin *RS*-mandelate, when measured with thermogravimetric analysis at a temperature in the range of from 25 to 200 °C and a heating rate of 10 K/min.
138) The crystalline omarigliptin *RS*-mandelate according to any one of the items 123 to 136, characterized by showing a weight loss of 0.4 weight% or less, based on the weight of the crystalline omarigliptin *RS*-mandelate, when measured with thermogravimetric analysis at a temperature in the range of from 25 to 200 °C and a heating rate of 10 K/min.
139) The crystalline omarigliptin *RS*-mandelate according to any one of the items 123 to 136, characterized by showing a weight loss of 0.3 weight% or less, based on the weight of the crystalline omarigliptin *RS*-mandelate, when measured with thermogravimetric analysis at a temperature in the range of from 25 to 200 °C and a heating rate of 10 K/min.
140) The crystalline omarigliptin *RS*-mandelate according to any one of the items 123 to 136, characterized by showing a weight loss of 0.2 weight% or less, based on the weight of the crystalline omarigliptin *RS*-mandelate, when measured with thermogravimetric analysis at a temperature in the range of from 25 to 200 °C and a heating rate of 10 K/min.
141) The crystalline omarigliptin *RS*-mandelate according to any one of the items 123 to 136, characterized by showing a weight loss of 0.1 weight% or less, based on the weight of the crystalline omarigliptin *RS*-mandelate, when measured with thermogravimetric analysis at a temperature in the range of from 25 to 200 °C and a heating rate of 10 K/min.
142) The crystalline omarigliptin *RS*-mandelate according to any one of the items 123 to 141, wherein the crystalline omarigliptin *RS*-mandelate is non-solvated.
143) Crystalline omarigliptin *RS*-mandelate according to anyone of items 123 to 142 characterized by showing a water uptake of at most 2.0 weight%, based on the weight of the crystalline omarigliptin *RS*-mandelate, when measured with gravimetric moisture sorption from about 0 to 95% relative humidity at a temperature of 25.0 ± 0.1 °C.
144) Crystalline omarigliptin *RS*-mandelate according to anyone of items 123 to 142 characterized by showing a water uptake of at most 1.5 weight%, based on the weight of the crystalline omarigliptin *RS*-mandelate, when measured with gravimetric moisture sorption from about 0 to 95% relative humidity at a temperature of 25.0 ± 0.1 °C.
145) Crystalline omarigliptin *RS*-mandelate according to anyone of items 123 to 142 characterized by showing a water uptake of at most 1.5 weight%, based on the weight of the crystalline omarigliptin *RS*-mandelate, when measured with gravimetric moisture sorption from about 0 to 95% relative humidity at a temperature of 25.0 ± 0.1 °C.
146) Crystalline omarigliptin *RS*-mandelate according to anyone of items 123 to 142 characterized by showing a water uptake of at most 0.8 weight% or, based on the weight of the crystalline omarigliptin *RS*-mandelate, when measured with gravimetric moisture sorption from about 0 to 95% relative humidity at a temperature of 25.0 ± 0.1 °C.
147) Crystalline omarigliptin *RS*-mandelate according to anyone of items 123 to 146, wherein the crystalline omarigliptin *RS*-mandelate is non-hygroscopic.
148) Crystalline omarigliptin *RS*-mandelate according to anyone of items 123 to 147, characterized by having a molar ratio of omarigliptin and *RS*-mandelic acid in the range of from 1.0: 0.8 to 1.0:1.2.
149) Crystalline omarigliptin *RS*-mandelate according to anyone of items 123 to 147, characterized by having a molar ratio of omarigliptin and *RS*-mandelic acid in the range of from 1.0: 0.9 to 1.0:1.1.
150) Crystalline omarigliptin *RS*-mandelate according to anyone of items 123 to 147, characterized by having a molar ratio of omarigliptin and *RS*-mandelic acid of 1.0: 1.0.
151) A process for the preparation of an omarigliptin acid addition salt, preferably of a crystalline omarigliptin acid addition salt according to any of the preceding items 1 to 150 comprising:
   (i) Providing the free base of omarigliptin in amorphous form, pseudo-amorphous form, crystalline form or as a mixture of two or more of these forms;
   (ii) Preparing a mixture comprising the omarigliptin provided in step (i) and a solvent system comprising at least one alcohol;
   (iii) Reacting the free base of omarigliptin comprised in the mixture provided in (ii) with an acid to obtain an acid addition salt of omarigliptin
   (iv) Separating the omarigliptin acid addition salt, preferably the crystalline omarigliptin acid addition salt of step (iii) from the reaction mixture.
152) The process according to item 151, wherein the omarigliptin acid addition salt is omarigliptin hydrochloride, omarigliptin fumarate, omarigliptin *R*-mandelate, omarigliptin S-mandelate or omarigliptin *RS*-mandelate.
153) The process according to any of items 151 to 152, wherein the omarigliptin acid addition salt is crystalline.
154) The process according to item 153, wherein the omarigliptin acid addition salt is crystalline omarigliptin hydrochloride, crystalline omarigliptin fumarate, crystalline omarigliptin *R*-mandelate, crystalline omarigliptin *S*-mandelate or crystalline omarigliptin *RS*-mandelate.
155) The process according to any of items 151 to 154, wherein in (i) the free base of omarigliptin is provided in amorphous form.
156) The process according to any of items 151 to 154, wherein in (i) the free base of omarigliptin is provided in crystalline form.
157) The process according to any of items 151 to 156, wherein in (ii) the at least one alcohol is an alcohol having 1 to 5 carbon atoms.
158) The process according to item 157, wherein in (ii) the alcohol is selected from the group consisting of methanol, ethanol, 1-propanol, 2-propanol, 1-butanol, 2-butanol, isobutanol, tert-butanol and isoamyl alcohol or mixtures of two or more thereof, preferably wherein the alcohol is selected from ethanol and 2-propanol, more preferably wherein the at least one alcohol is ethanol.
159) The process according to item 158, wherein the at least one alcohol is ethanol.
160) The process according to any of items 151 to 159, wherein in (ii) the mixture comprises omarigliptin in a concentration in the range of from about 5 to 50 g/L, more preferably from about 10 to 40 g/ and most preferably from about 15 to 35 g/L solvent.
161) The process according to any of items 151 to 160, wherein step (ii) comprises dissolving, more preferably completely dissolving, the omarigliptin free base provided in (i) in the solvent system comprising the at least one alcohol.
162) The process according to any of items 151 to 161, wherein in (iii) the acid is selected from the group consisting of hydrochloric acid, fumaric acid, *R*-mandelic acid, S-mandelic acid and *RS*-mandelic acid.
163) The process according to item 162, wherein in (iii) the acid is hydrochloric acid.
164) The process according to item 163, wherein in (iii) the acid is aqueous hydrochloric acid having a concentration in the range of from about 0.1 to 37 weight%, preferably from about 9 to 37 weight%, more preferably from about 18 to 37 weight%.
165) The process according to item 164, wherein in (iii) the acid is concentrated hydrochloric acid having a concentration of about 37 weight%.
166) The process of any of items 151 to 165, wherein in (iii) the molar ratio of the omarigliptin free base and the acid is in the range of from about 1.0:0.8 to 1.0:1.2, preferably from about 1.0:0.9 to 1.0:1.1, more preferably of about 1.0 : 1.0.
167) The process of any of items 151 to 166, wherein in step (iv) the separated omarigliptin acid addition salt is in amorphous or in crystalline form.
168) The process of item 167, wherein in (iv) the separated omarigliptin acid addition salt is crystalline.
169) The process of item 168, wherein in (iv) the separated omarigliptin acid addition salt is crystalline omarigliptin hydrochloride, crystalline omarigliptin fumarate, crystalline omarigliptin *R*-mandelate, crystalline omarigliptin *S*-mandelate or crystalline omarigliptin *RS*-mandelate.
170) The process of any of items 168 or 169, wherein step (iv) further comprises (iv.1) crystallizing the omarigliptin acid addition salt obtained in (iii) to obtain the crystalline acid addition salt of omarigliptin.
171) The process of item 170, wherein in (iv.1) crystallization is carried out by cooling the reaction mixture obtained in step (iii) to a temperature of about 40 °C or less, preferably of about 30 °C or less, preferably of about 20 °C or less, more preferably of about 10 °C or less.
172) The process of item 170, wherein in (iv.1) crystallization is carried out by first cooling the mixture to about room temperature and then further cooling the mixture to a temperature in the range of from about 2 to 8 °C.
173) The process of any of items 170 to 172, wherein step (iv.1) further comprises adding seed crystals to the reaction mixture.
174) The process of any of items 151 to 173, further comprising
   (v) optionally collecting the non-hygroscopic acid addition salt of omarigliptin, preferably the crystalline non-hygroscopic acid addition salt of omarigliptin of step (iv).
175) The process of any of items 151 to 174, further comprising
   (vi) optionally washing the non-hygroscopic acid addition salt of omarigliptin, preferably the crystalline non-hygroscopic acid addition salt of omarigliptin with a suitable solvent or solvent mixture.
176) The process of item 175, wherein the suitable solvent or solvent mixture comprises a water-miscible organic solvent and water.
177) The process of item 176, wherein the water-miscible organic solvent is selected from the group consisting of alcohols, ketones, nitriles, cyclic ethers or mixtures of two or more of them.
178) The process of item 177, wherein the suitable solvent or solvent mixture comprises an aqueous alcohol.
179) The process of item 178, wherein the alcohol is methanol, ethanol, 1-propanol, 2-propanol or a mixture of two or more thereof.
180) The process of item 175, wherein the suitable solvent is water.
181) The process of any of items 151 to 181, further comprising
   (vii) optionally drying the non-hygroscopic acid addition salt of omarigliptin, preferably the crystalline non-hygroscopic acid addition salt of omarigliptin.
182) The process of item 181, wherein drying in step (vii) is carried out at a temperature of about 100 °C or less, preferably of about 80 °C or less, preferably of about 60 °C or less, more preferably of about 40 °C or less.
183) The process of item 181 or 182, wherein drying in step (vii) is carried out at room temperature.
184) The process of any of items 181 to 183, wherein drying in step (vii) is performed for a period of time in the range of from about 1 to 72 hours, preferably from about 2 to 48 hours, preferably from about 4 to 24 hours, more preferably from about 6 to 18 hours.
185) The process of any of items 181 to 184, wherein drying in step (vii) is performed under vacuum at about 100 mbar or less, preferably at about 50 mbar or less, preferably at about 30 mbar or less, more preferably at about 20 mbar or less
186) Use of the non-hygroscopic omarigliptin acid addition salt according of any of items 1 to 150, preferably of the crystalline non-hygroscopic omarigliptin acid addition salt according to any of items 6 or 17 to 150 for the preparation of a pharmaceutical composition.
187) A pharmaceutical composition comprising an effective amount of the non-hygroscopic omarigliptin acid addition salt as defined in anyone of items 1 to 150, preferably of the crystalline non-hygroscopic omarigliptin acid addition salt according to any of items 6 or 17 to 150 and at least one pharmaceutically acceptable excipient.
188) The pharmaceutical composition according to item 187 for use as a medicament.
189) The pharmaceutical composition according to item 187 or 188 for use in the treatment of a condition selected from diabetes type 2, obesity and high blood pressure.
190) A non-hygroscopic omarigliptin acid addition salt obtainable or obtained by the process according to any of items 151 to 185.
191) The non-hygroscopic omarigliptin acid addition salt of item 190, wherein the salt is crystalline.
192) The non-hygroscopic omarigliptin acid addition salt of item 191, wherein the salt is crystalline omarigliptin hydrochloride, crystalline omarigliptin fumarate, crystalline omarigliptin *R*-mandelate, crystalline omarigliptin *S*-mandelate or crystalline omarigliptin *RS*-mandelate.

The following non-limiting examples are illustrative for the disclosure.

### EXAMPLES

### Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-theta with 40s per step (255 channels) in the angular range of 2° to 40° 2-theta at ambient conditions. A typical precision of the 2-theta values is in the range of ± 0.2° 2-theta, preferably of ± 0.1° 2-theta. Thus, the diffraction peak of crystalline omarigliptin hydrochloride that appears for example at 18.7° 2-theta can appear in the range of from 18.5 to 18.9° 2-theta, preferably in the range of from 18.6 to 18.8 ° 2-theta on most X-ray diffractometers under standard conditions.

### Fourier transform infrared spectroscopy

Fourier transform infrared spectroscopy (FTIR) was performed with a MKII Golden Gate™ Single Reflection Diamond ATR (attenuated total reflection) cell with a Bruker Tensor 27 FTIR spectrometer with 4 cm⁻¹ resolution at ambient conditions. To record a spectrum a spatula tip of the sample was applied to the surface of the diamond in powder form. Then the sample was pressed onto the diamond with a sapphire anvil and the spectrum was recorded. A spectrum of the clean diamond was used as background spectrum. A typical precision of the wavenumber values is in the range of about ± 2 cm⁻¹. Thus, for example the infrared peak of crystalline omarigliptin hydrochloride that appears at 1515 cm⁻¹ can appear between 1513 and 1517 cm⁻¹ on most infrared spectrometers under standard conditions.

### Differential scanning calorimetry

DSC was performed on a Mettler Polymer DSC R instrument. The samples (3.16 mg HC1 salt, 1.46 mg *R*-mandelate, 1.55 mg S-mandelate, 1.72 mg *RS*-mandelate and 2.07 mg fumarate respectively) were heated in a 40 microL aluminum pan with pierced aluminum lid from 25 to 250 °C at a rate of 10 K/min. Nitrogen (purge rate 50 mL/min) was used as purge gas.

### Thermogravimetric analysis

TGA was performed on a Mettler TGA/DSC 1 instrument. The samples (7.06 mg HC1 salt, 2.23 mg *R*-mandelate, 5.13 mg S-mandelate, 7.70 mg *RS*-mandelate and 5.15 mg fumarate respectively) were heated in a 100 microL aluminum pan closed with an aluminum lid. The lid was automatically pierced at the beginning of the measurement. The samples were heated from 25 to 250 °C at a rate of 10 K/min. Nitrogen (purge rate 50 mL/min) was used as purge gas.

### Gravimetric moisture sorption

Moisture sorption isotherms were recorded with an SPSx-1µ moisture sorption analyzer (proUmid, Ulm). The measurement cycle was started at ambient relative humidity (RH) of 35% and first decreased to 3% RH and 0% RH. Then RH was increased from 0% to 10% in 5% steps, from 10 to 90% in 10% steps and from 90 to 95% in a 5% step. Subsequently, RH was decreased from 95% to 90% in a 5% step and from 90% to 0% in 10% steps. Finally, RH was increased from 0% to 10% in a 10% step and to ambient RH of 35% in one step. The time per step was set to a minimum of 2 hours and a maximum of 5 hours. If an equilibrium condition with a constant mass of ± 0.01% within 1 hour was reached before the maximum time for all examined samples, the sequential humidity step was applied before the maximum time of 5 hours. If no equilibrium was achieved, the consecutive humidity step was applied after the maximum time of 5 hours. The temperature was 25 ± 0.1°C.

### Example 1: Preparation of crystalline omarigliptin hydrochloride

Omarigliptin (1.0 g, 2.5 mmol, for example prepared according to the procedure disclosed in WO 2010/056708 A1) was suspended in absolute ethanol (30 mL) at RT. After heating to reflux temperature a clear solution was obtained and concentrated hydrochloric acid (37 weight% aqueous solution, 220 microL, 2,65mmol) was added to the hot solution. Subsequently, the mixture was allowed to cool to room temperature in order to initiate crystallization. The resulting suspension was further stored at a temperature of about 2 to 8 °C for 20 hours. The crystals were collected by filtration and dried at RT under vacuum (30 mbar) for 3 hours to yield 0.6 g omarigliptin hydrochloride.

### Yield: 55% of theory

The solid state properties of the obtained material were investigated by means of PXRD, FTIR, DSC, TGA and GMS. The results are discussed in the section below:
The PXRD of crystalline omarigliptin hydrochloride is displayed in figure 1 herein. A reflection list and the corresponding relative intensities are provided in table 1. The most characteristic reflections are marked in bold print, wherein each of these reflections alone or any combination thereof can be used to inevitably characterize crystalline omarigliptin hydrochloride of the present invention.

**Table 1: Reflection list and corresponding relative intensities of crystalline omarigliptin hydrochloride in the range of from 2.0 to 30.0° 2-theta; the most characteristic reflections are marked in bold print;**

| **Angle** **[± 0.2 °2-Theta]** | **Relative Intensity** **[%]** | **Angle** **[ ± 0.2 °2-Theta]** | **Relative Intensity** **[%]** |
|---|---|---|---|
| **4.8** | **9** | 19.2 | 2 |
| **8.7** | **14** | **20.3** | **17** |
| 9.6 | 2 | **20.8** | **25** |
| 9.7 | 1 | **21.0** | **51** |
| **10.1** | **14** | 21.5 | 2 |
| 13.2 | 2 | **24.2** | **16** |
| 14.4 | 4 | 26.3 | 5 |
| **16.5** | **16** | **26.9** | 14 |
| 17.2 | 5 | 27.7 | 5 |
| **17.4** | **18** | 28.0 | 3 |
| 17.9 | 1 | 28.5 | 3 |
| **18.7** | **100** | 29.9 | 4 |

The FTIR spectrum of crystalline omarigliptin hydrochloride is displayed in figure 2 herein. A peak list is provided in table 2. The most characteristic peaks are marked in bold print, wherein each of these peaks alone or any combination thereof can be used to inevitably characterize crystalline omarigliptin hydrochloride of the present invention.

**Table 2: FTIR peak list of crystalline omarigliptin hydrochloride in the range of from 4000 to 600 cm⁻¹; the most characteristic peaks are marked in bold print;**

| **Wavenumber** **[cm⁻¹]** | **Wavenumber** **[cm⁻¹]** | **Wavenumber** **[cm⁻¹]** | **Wavenumber** **[cm⁻¹]** |
|---|---|---|---|
| 3025 | 1496 | 1209 | 839 |
| **2849** | 1469 | 1174 | 821 |
| **2794** | 1433 | 1141 | 809 |
| 2694 | 1414 | 1097 | 771 |
| 2653 | 1394 | 1071 | 731 |
| 2566 | 1360 | 1058 | 689 |
| 2528 | **1326** | 1022 | 657 |
| 2451 | 1304 | 980 | |
| 1593 | **1274** | 921 | |
| **1515** | 1245 | 863 | |

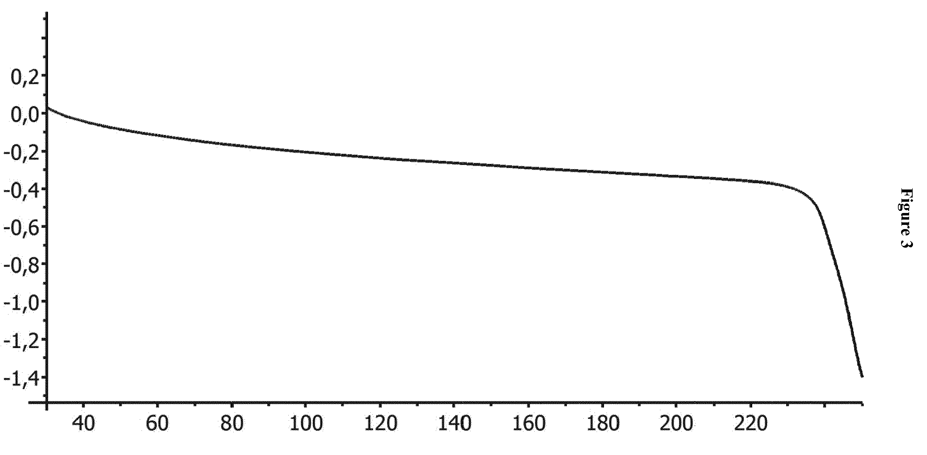

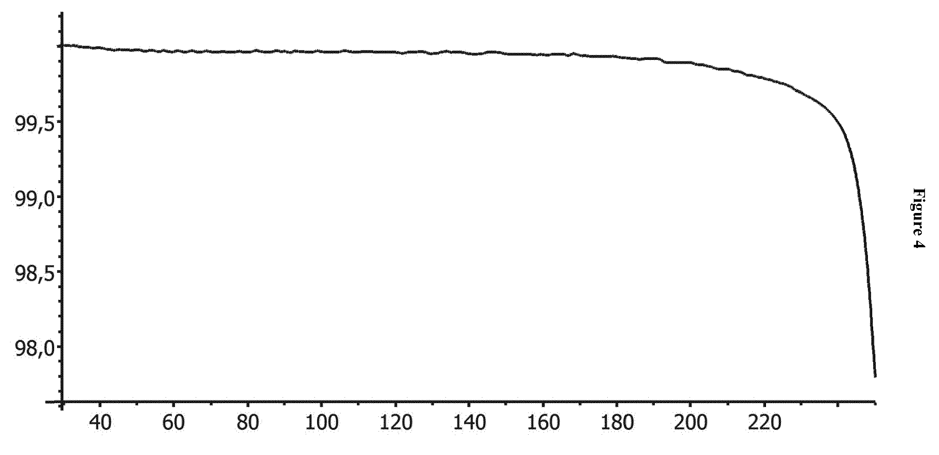

The DSC thermogram of crystalline omarigliptin hydrochloride which is also displayed in figure 3 herein shows no thermal event until it starts to decompose at a temperature above about 200 °C, which is expressed by an exothermic signal. Therefore, crystalline omarigliptin hydrochloride according to the present invention exhibits no melting point. The TGA curve of crystalline omarigliptin hydrochloride which is also displayed in figure 4 herein shows only a slight weight loss of less than about 0.2 weight% up to a temperature of about 200 °C.

The GMS curve of crystalline omarigliptin hydrochloride, shows almost no interaction with water vapor. The water uptake at 25.0 ± 0.1 °C observed in the first sorption cycle from 0 to 95% relative humidity is only about 2.0%. In addition, no hysteresis between the sorption and desorption curve can be observed, which is a hint that no structural changes occurred during the experiment. PXRD performed after the GMS experiment confirmed the presence of the initially applied crystalline salt form.

From the thermal investigations and the GMS experiment it can be concluded that the crystalline omarigliptin hydrochloride of the present invention is an anhydrous, non-solvated and non-hygroscopic form, which exhibits no melting point but decomposes at a temperature beyond about 200 °C.

### Example 2: Preparation of crystalline omarigliptin fumarate

Omarigliptin (250 mg, 0.6 mmol, for example prepared according to the procedure disclosed in WO 2010/056708 A1) was suspended in absolute ethanol (7.5 mL) at RT. After heating to reflux temperature a clear solution was obtained. A solution of fumaric acid (76.5 mg, 0.6 mmol) in absolute ethanol (1 mL) was added to the hot solution and subsequently the mixture was allowed to cool to room temperature in order to initiate crystallization. The resulting suspension was further stored at a temperature of about 2 to 8 °C for 20 hours. The crystals were collected by filtration and dried at RT under vacuum (30 mbar) for 3 hours to yield 250 mg omarigliptin fumarate.

### Yield: 78% of theory

The solid state properties of the obtained material were investigated by means of PXRD, FTIR, DSC, TGA and GMS.

The PXRD of crystalline omarigliptin fumarate is displayed in figure 5 herein. A reflection list and the corresponding relative intensities are provided in table 3. The most characteristic reflections are marked in bold print, wherein each of these reflections alone or any combination thereof can be used to inevitably characterize crystalline omarigliptin fumarate of the present invention.

**Table 3: Reflection list and corresponding relative intensities of crystalline omarigliptin fumarate in the range of from 2.0 to 30.0° 2-theta; the most characteristic reflections are marked in bold print;**

| **Angle** **[ ± 0.2 °2-Theta]** | **Relative Intensity** **[%]** | **Angle** **[± 0.2 °2-Theta]** | **Relative Intensity** **[%]** |
|---|---|---|---|
| 6.5 | 9 | **20.4** | 40 |
| **6.9** | 26 | **20.6** | 37 |
| 8.9 | 20 | 20.8 | 30 |
| 10.8 | 30 | **21.2** | 88 |
| **11.2** | 3 | 21.5 | 5 |
| 13.1 | 7 | **22.1** | 70 |
| **13.3** | 8 | 22.5 | 72 |
| 13.8 | 95 | 23.1 | 46 |
| **14.4** | 11 | 23.3 | 14 |
| **15.2** | 26 | 23.6 | 35 |
| **15.6** | 5 | 24.0 | 3 |
| **16.2** | 38 | 24.4 | 28 |
| **16.8** | 15 | 24.6 | 29 |
| **17.1** | 21 | 24.8 | 77 |
| **17.5** | 55 | 25.4 | 29 |
| **17.8** | 38 | 26.1 | 22 |
| **18.2** | 46 | 26.5 | 29 |
| **18.4** | 6 | 28.5 | 3 |
| **18.9** | 21 | 29.6 | 4 |
| 19.1 | 7 | 30.0 | 5 |
| **19.7** | 100 | | |

The FTIR spectrum of crystalline omarigliptin fumarate is displayed in figure 6 herein. A peak list is provided in table 6. The most characteristic peaks are marked in bold print, wherein each of these peaks alone or any combination thereof can be used to inevitably characterize crystalline omarigliptin fumarate of the present invention.

**Table 6: FTIR peak list of crystalline omarigliptin fumarate in the range of from 4000 to 600 cm⁻¹; the most characteristic peaks are marked in bold print;**

| **Wavenumber** **[cm⁻¹]** | **Wavenumber** **[cm⁻¹]** | **Wavenumber** **[cm⁻¹]** | **Wavenumber** **[cm⁻¹]** |
|---|---|---|---|
| **3301** | 1496 | 1182 | 864 |
| **3088** | 1464 | **1165** | 834 |
| 3008 | 1421 | 1127 | 810 |
| 2927 | 1392 | 1102 | 772 |
| 2876 | **1359** | 1076 | 738 |
| 2802 | 1304 | 1061 | 689 |
| 1873 | 1282 | 974 | 689 |
| 1650 | 1268 | 936 | |
| 1610 | 1250 | 926 | |
| **1573** | 1207 | 906 | |

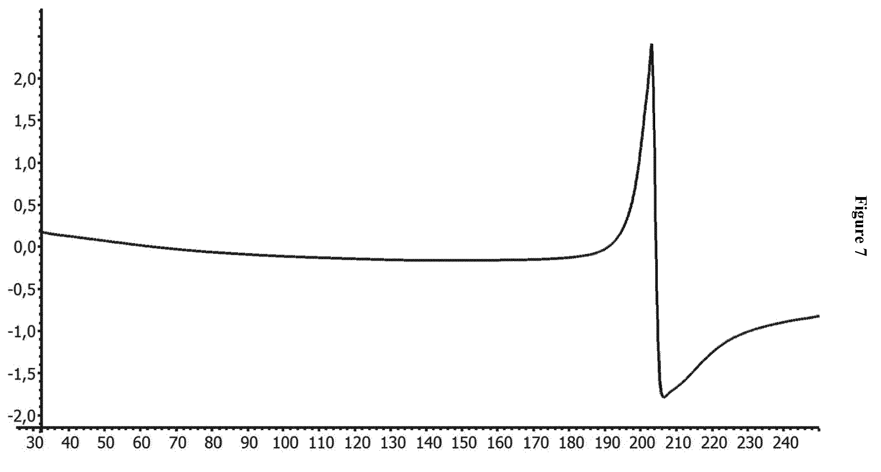

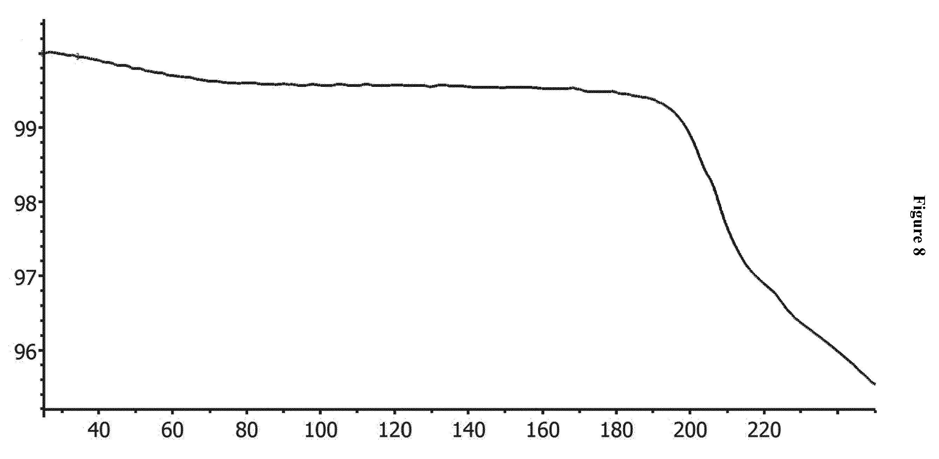

The DSC thermogram of crystalline omarigliptin fumarate which is also displayed in figure 7 herein shows no thermal event until the salt melts, which is indicated by an endotherm having an onset temperature of about 175.1 °C, a peak maximum at about 201.2 °C and a heat of fusion of about 59.5 J/g. Immediately after melting decomposition occurs. The TGA curve of crystalline omarigliptin fumarate which is also displayed in figure 8 herein shows a minor weight loss of about 0.3 weight% from the beginning of the measurement until a temperature of about 80 °C, which is most likely due to the release of residual ethanol and/ or water. Decomposition starts at approximately 200 °C.

The GMS curve of crystalline omarigliptin fumarate shows almost no interaction with water vapor. The water uptake at 25.0 ± 0.1 °C observed in the first sorption cycle from 0 to 95% relative humidity is only about 1.4%. In addition, no hysteresis between the sorption and desorption curve can be observed, which is a hint that no structural changes occurred during the experiment. PXRD performed after the GMS experiment confirmed the presence of the initially applied crystalline salt form.

From the thermal investigations and the GMS experiment it can be concluded that crystalline omarigliptin fumarate of the present invention is an anhydrous, non-solvated and non-hygroscopic form, which melts at a temperature in the range of from about 175 to 201 °C.

### Example 3: Preparation of crystalline omarigliptin R-mandelate

Omarigliptin (1.0 g, 2.5 mmol, for example prepared according to the procedure disclosed in WO 2010/056708 A1) was suspended in absolute ethanol (30 mL) at RT. After heating to reflux temperature a clear solution was obtained. A solution of *R*-mandelic acid (0.4 g, 2.6 mmol) in absolute ethanol (5 mL) was added to the hot solution and subsequently the mixture was allowed to cool to room temperature in order to initiate crystallization. The resulting suspension was further stored at a temperature of about 2 to 8 °C for 20 hours. The crystals were collected by filtration and dried at RT under vacuum (30 mbar) for 3 hours to yield 1.3 g omarigliptin *R*-mandelate.

### Yield: 94% of theory

The solid state properties of the obtained material were investigated by means of PXRD, FTIR, DSC, TGA and GMS.

The powder X-ray diffractogram of crystalline omarigliptin *R*-mandelate is displayed in figure 9 herein. A reflection list and the corresponding relative intensities are provided in table 5. The most characteristic reflections are marked in bold print, wherein each of these reflections alone or any combination thereof can be used to inevitably characterize crystalline omarigliptin *R-*mandelate of the present invention.

**Table 5: Reflection list and corresponding relative intensities of crystalline omarigliptin R-mandelate in the range of from 2.0 to 30.0° 2-theta; the most characteristic reflections are marked in bold print;**

| **Angle** **[± 0.2 °2-Theta]** | **Relative Intensity** **[%]** | **Angle** [**± 0.2 °2-Theta]** | **Relative Intensity** **[%]** |
|---|---|---|---|
| 5.1 | 10 | **21.9** | 89 |
| **6.3** | 88 | 22.2 | 30 |
| **7.1** | 42 | 22.8 | 45 |
| 9.0 | 3 | **23.2** | 66 |
| **10.6** | 16 | 23.4 | 59 |
| **12.6** | 39 | 24.5 | 4 |
| 13.2 | 11 | 24.7 | 5 |
| 14.7 | 4 | 25.0 | 19 |
| **15.1** | 55 | 25.4 | 21 |
| **15.3** | 59 | 25.7 | 6 |
| 17.4 | 15 | 26.1 | 4 |
| **17.9** | 100 | 26.4 | 9 |
| 18.5 | 12 | 26.9 | 8 |
| 18.9 | 20 | 27.3 | 9 |
| 20.0 | 8 | **28.0** | 41 |
| 20.5 | 47 | 28.6 | 2 |
| **20.8** | 48 | 29.7 | 4 |
| 21.4 | 7 | | |

The FTIR spectrum of crystalline omarigliptin *R*-mandelate is displayed in figure 10 herein. A peak list is provided in table 6. The most characteristic peaks are marked in bold print, wherein each of these peaks alone or any combination thereof can be used to inevitably characterize crystalline omarigliptin *R*-mandelate of the present invention.

**Table 6: FTIR peak list of crystalline omarigliptin R-mandelate in the range of from 4000 to 600 cm⁻¹; the most characteristic peaks are marked in bold print;**

| **Wavenumber** **[cm⁻¹]** | **Wavenumber** **[cm⁻¹]** | **Wavenumber** **[cm⁻¹]** | **Wavenumber** **[cm⁻¹]** |
|---|---|---|---|
| **3390** | 1452 | 1144 | 818 |
| 3138 | 1418 | 1131 | 803 |
| 3082 | **1371** | 1110 | 774 |
| 3031 | 1344 | 1096 | 741 |
| 2935 | 1307 | 1077 | 729 |
| 2885 | 1281 | 1064 | 696 |
| 2804 | 1263 | **1037** | 658 |
| **1636** | 1244 | 979 | 619 |
| 1596 | 1214 | 928 | |
| 1556 | 1183 | 872 | |
| **1487** | 1166 | 838 | |

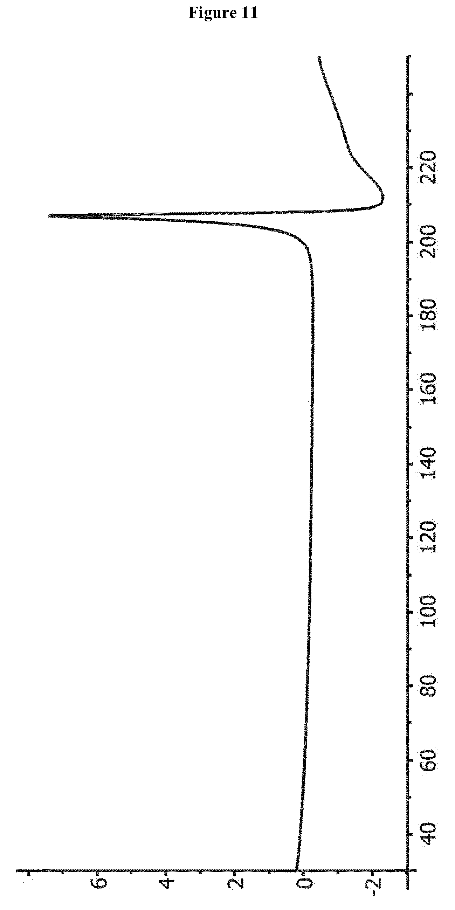

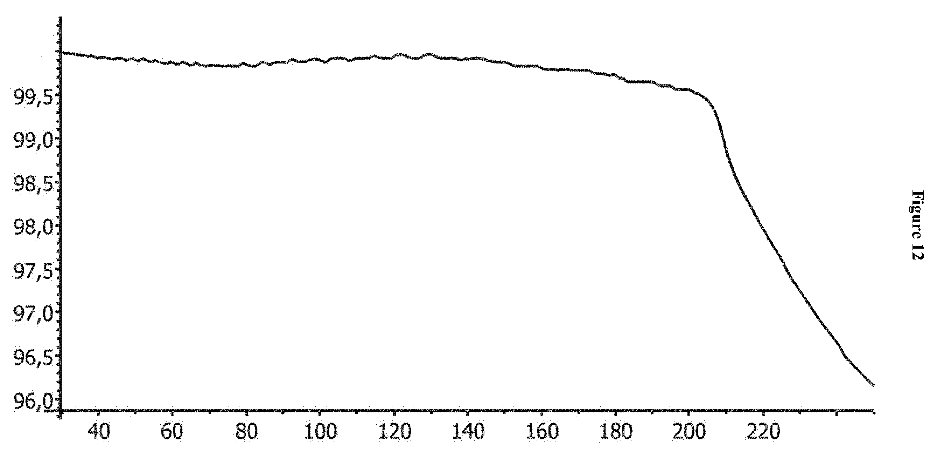

The DSC thermogram of crystalline omarigliptin *R*-mandelate which is also displayed in figure 11 herein shows no thermal event until it melts, which is indicated by an endotherm having an onset temperature of about 204.2 °C, a peak maximum at about 206.3 °C and a heat of fusion of about 123.16 J/g. Immediately after melting decomposition occurs. The TGA curve of crystalline omarigliptin *R*-mandelate which is also displayed in figure 12 herein shows no significant weight loss until decomposition starts at approximately 200 °C.

The GMS curve of crystalline omarigliptin *R*-mandelate shows almost no interaction with water vapor. The water uptake at 25.0 ±0.1 °C observed in the first sorption cycle from 0 to 95% relative humidity is only about 0.7%. In addition, no hysteresis between the sorption and desorption curve can be observed, which is a hint that no structural changes occurred during the experiment. PXRD performed after the GMS experiment confirmed the presence of the initially applied crystalline salt form.

From the thermal investigations and the GMS experiment it can be concluded that crystalline omarigliptin *R*-mandelate of the present invention is an anhydrous, non-solvated and non-hygroscopic crystalline form, which melts at a temperature in the range of from about 204 to 206 °C.

### Example 4: Preparation of crystalline omarigliptin S-mandelate

Omarigliptin (250 mg, 0.6 mmol, for example prepared according to the procedure disclosed in WO 2010/056708 A1) was suspended in absolute ethanol (7.5 mL) at RT. After heating to reflux temperature a clear solution was obtained. A solution of S-mandelic acid (100 mg, 0.7 mmol) in absolute ethanol (1 mL) was added to the hot solution and subsequently the mixture was allowed to cool to room temperature in order to initiate crystallization. The resulting suspension was further stored at a temperature of about 2 to 8 °C for 20 hours. The crystals were collected by filtration and dried at RT under vacuum (30 mbar) for 3 hours to yield 300 mg omarigliptin *S*-mandelate.

### Yield: 87% of theory

The solid state properties of the obtained material were investigated by means of PXRD, FTIR, DSC, TGA and GMS.

The powder X-ray diffractogram of crystalline omarigliptin S-mandelate is displayed in figure 13 herein. A reflection list and the corresponding relative intensities are provided in table 7. The most characteristic reflections are marked in bold print, wherein each of these reflections alone or any combination thereof can be used to inevitably characterize crystalline omarigliptin *S-*mandelate of the present invention.

**Table 7: Reflection list and corresponding relative intensities of crystalline omarigliptin S-mandelate in the range of from 2.0 to 30.0° 2-theta; the most characteristic reflections are marked in bold print;**

| **Angle** | **Relative Intensity** | **Angle** | **Relative Intensity** |
|---|---|---|---|
| **[± 0.2 °2-Theta]** | **[%]** | **[± 0.2 °2-Theta]** | **[%]** |
| 6.2 | 15 | **21.0** | 45 |
| **7.1** | 31 | 21.4 | 34 |
| **8.8** | 33 | 21.9 | 25 |
| 10.9 | 16 | **22.8** | 27 |
| 11.9 | 16 | 23.2 | 3 |
| **14.9** | 22 | 23.7 | 8 |
| 15.4 | 4 | 25.2 | 13 |
| 15.9 | 24 | 25.6 | 29 |
| **16.3** | 44 | 25.9 | 25 |
| 17.0 | 5 | 26.2 | 3 |
| **17.4** | 58 | 26.8 | 7 |
| **18.2** | 60 | 27.9 | 2 |
| **18.6** | 100 | 28.1 | 4 |
| 18.8 | 7 | 28.7 | 2 |
| 19.2 | 9 | 29.2 | 4 |
| 19.6 | 12 | 29.7 | 3 |
| **20.7** | 65 | | |

The FTIR spectrum of crystalline omarigliptin S-mandelate is displayed in figure 14 herein. A peak list is provided in table 8. The most characteristic peaks are marked in bold print, wherein each of these peaks alone or any combination thereof can be used to inevitably characterize crystalline omarigliptin S-mandelate of the present invention.

**Table 8: FTIR peak list of crystalline omarigliptin S-mandelate in the range of from 4000 to 600 cm⁻¹; the most characteristic peaks are marked in bold print;**

| **Wavenumber** **[cm⁻¹]** | **Wavenumber** **[cm⁻¹]** | **Wavenumber** **[cm⁻¹]** | **Wavenumber** **[cm⁻¹]** |
|---|---|---|---|
| **3292** | 1454 | **1218** | 849 |
| 3134 | **1432** | 1172 | 818 |
| 3027 | 1413 | 1143 | 806 |
| 3002 | 1418 | 1103 | 769 |
| 2924 | **1365** | 1063 | 732 |
| 2854 | 1320 | 1044 | 695 |
| 2798 | 1284 | 969 | 656 |
| **1544** | 1268 | 921 | 618 |
| 1498 | 1247 | 866 | |

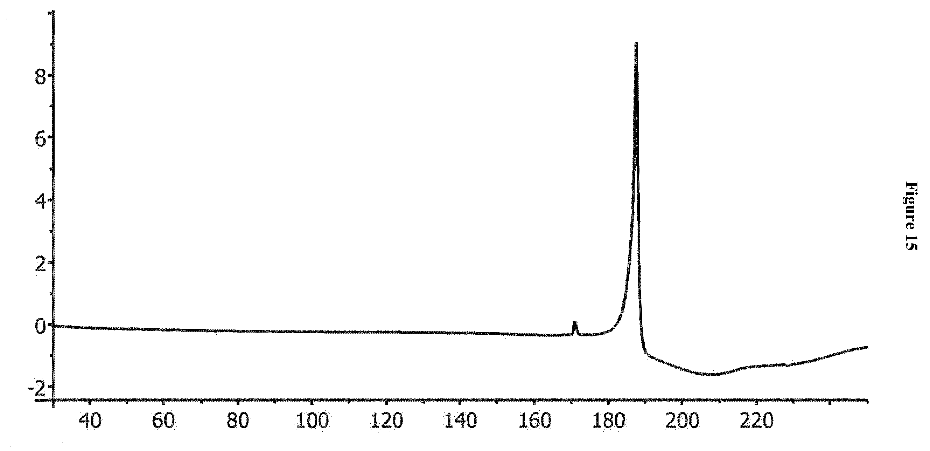

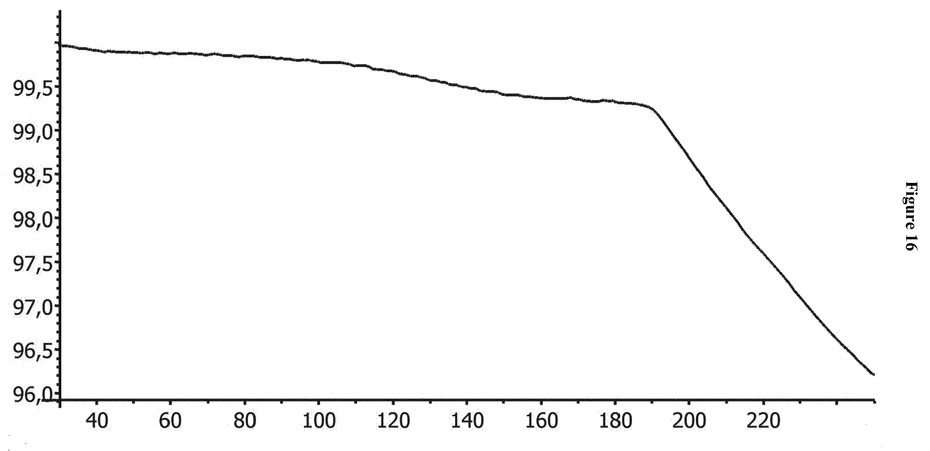

The DSC thermogram of crystalline omarigliptin *S*-mandelate which is also displayed in figure 15 herein shows no thermal event until it melts, which is indicated by an endotherm having an onset temperature of about 185.5 °C, a peak maximum at about 186.9 °C and a heat of fusion of about 138.89 J/g. Immediately after melting decomposition occurs. The TGA curve of crystalline omarigliptin S-mandelate which is also displayed in figure 16 herein shows a minor weight loss of about 0.5 weight% at a temperature in the range of from about 80 °C to 160 °C, which is most likely due to the release of residual ethanol and/ or water.

The GMS curve of crystalline omarigliptin S-mandelate shows almost no interaction with water vapor. The water uptake at 25.0 ±0.1 °C observed in the first sorption cycle from 0 to 95% relative humidity is only about 1.0%. In addition, no hysteresis between the sorption and desorption curve can be observed, which is a hint that no structural changes occurred during the experiment. PXRD performed after the GMS experiment confirmed the presence of the initially applied crystalline salt form.

From the thermal investigations and the GMS experiment it can be concluded that crystalline omarigliptin S-mandelate of the present invention is an anhydrous, non-solvated and non-hygroscopic crystalline form, which melts at a temperature in the range of from about 186 to 187 °C.

### Example 5: Preparation of crystalline omarigliptin RS-mandelate

Omarigliptin (150 mg, 0.4 mmol, for example prepared according to the procedure disclosed in WO 2010/056708 A1) was suspended in absolute ethanol (7.5 mL) at RT. After heating to reflux temperature a clear solution was obtained. A solution of *RS*-mandelic acid (100 mg, 0.7 mmol) in absolute ethanol (1 mL) was added to the hot solution and subsequently the mixture was allowed to cool to room temperature in order to initiate crystallization. The resulting suspension was further stored at a temperature of about 2 to 8 °C for 20 hours. The crystals were collected by filtration and dried at RT under vacuum (30 mbar) for 3 hours to yield 140mg omarigliptin *RS*-mandelate.

### Yield: 68% of theory

The solid state properties of the obtained material were investigated by means of PXRD, FTIR, DSC, TGA and GMS.

The powder X-ray diffractogram of crystalline omarigliptin *RS*-mandelate is displayed in figure 17 herein. A reflection list and the corresponding relative intensities are provided in table 9. The most characteristic reflections are marked in bold print, wherein each of these reflections alone or any combination thereof can be used to inevitably characterize crystalline omarigliptin *RS-*mandelate of the present invention.

**Table 9: Reflection list and corresponding relative intensities of crystalline omarigliptin RS-mandelate in the range of from 2.0 to 30.0° 2-theta; the most characteristic reflections are marked in bold print;**

| **Angle** **[± 0.2 °2-Theta]** | **Relative Intensity** **[%]** | **Angle** **[ ± 0.2 °2-Theta]** | **Relative Intensity** **[%]** |
|---|---|---|---|
| 5.1 | 7 | 17.9 | 54 |
| **6.3** | 100 | 18.2 | 43 |
| **7.1** | 32 | **18.6** | 72 |
| **8.8** | 20 | 19.1 | 7 |
| 10.7 | 11 | 19.6 | 8 |
| 10.9 | 12 | 20.3 | 26 |
| 11.8 | 9 | **20.7** | 54 |
| **12.7** | 52 | 21.0 | 29 |
| 13.2 | 6 | **21.7** | 42 |
| 14.9 | 12 | 22.8 | 38 |
| 15.2 | 25 | **23.3** | 42 |
| **15.4** | 30 | 25.1 | 12 |
| 15.9 | 20 | 25.6 | 18 |
| **16.3** | 43 | 25.9 | 14 |
| 17.4 | 39 | 26.7 | 11 |
| 17.7 | 47 | 27.9 | 17 |

The FTIR spectrum of crystalline omarigliptin *RS*-mandelate is displayed in figure 18 herein. A peak list is provided in table 10. The most characteristic peaks are marked in bold print, wherein each of these peaks alone or any combination thereof can be used to inevitably characterize crystalline omarigliptin *RS*-mandelate of the present invention.

**Table 10: FTIR peak list of crystalline omarigliptin S-mandelate in the range of from 4000 to 600 cm⁻¹; the most characteristic peaks are marked in bold print;**

| **Wavenumber** **[cm⁻¹]** | **Wavenumber** **[cm⁻¹]** | **Wavenumber** **[cm⁻¹]** | **Wavenumber** **[cm⁻¹]** |
|---|---|---|---|
| **3292** | 1454 | **1218** | 849 |
| 3134 | **1432** | 1172 | 818 |
| 3027 | 1413 | 1143 | 806 |
| 3002 | 1418 | 1103 | 769 |
| 2924 | **1365** | 1063 | 732 |
| 2854 | 1320 | 1044 | 695 |
| 2798 | 1284 | 969 | 656 |
| **1544** | 1268 | 921 | 618 |
| 1498 | 1247 | 866 | |

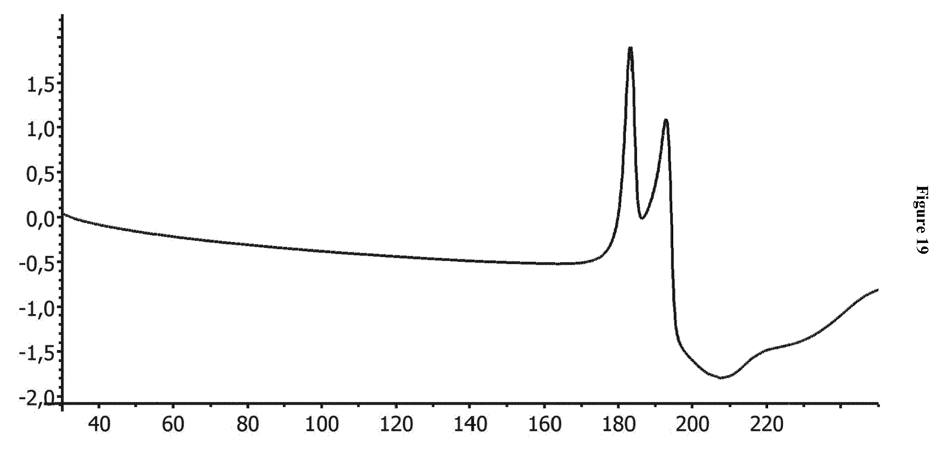

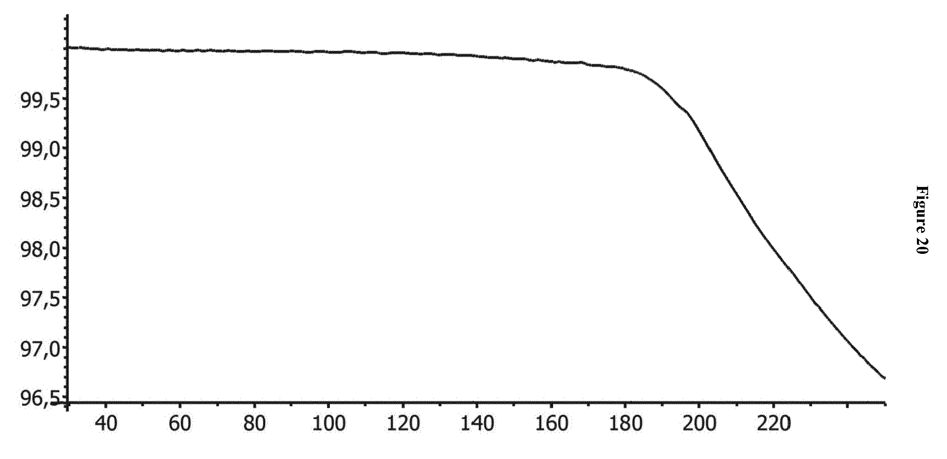

The DSC thermogram of crystalline omarigliptin *RS*-mandelate which is also displayed in figure 19 herein shows no thermal event until it melts, which is indicated by a double peak with a first peak maximum at about 186.9 °C and a second peak maximum at about 192.6°C. Immediately, after melting decomposition occurs. The TGA curve of crystalline omarigliptin *RS*-mandelate which is also displayed in figure 20 herein shows no significant weight loss until decomposition starts at approximately 180 °C.

The GMS curve of crystalline omarigliptin *RS*-mandelate shows almost no interaction with water vapor. The water uptake at 25.0 ±0.1 °C observed in the first sorption cycle from 0 to 95% relative humidity is only about 0.8%. In addition, no hysteresis between the sorption and desorption curve can be observed, which is a hint that no structural changes occurred during the experiment. PXRD performed after the GMS experiment confirmed the presence of the initially applied crystalline salt form.

From the thermal investigations and the GMS experiment it can be concluded that crystalline omarigliptin *RS*-mandelate of the present invention is an anhydrous, non-solvated and non-hygroscopic crystalline form, which melts at a temperature in the range of from about 187 to 193 °C.

## Claims

1. An acid addition salt of omarigliptin, wherein the omarigliptin acid addition salt shows a water uptake of at most 2.0 weight%, based on the weight of the omariglipitin acid addition salt, when measured with gravimetric moisture sorption at a relative humidity in the range of from 0 to 95% and a temperature of 25.0 ± 0.1 °C.

2. The omarigliptin acid addition salt according to claim 1, wherein the omarigliptin acid addition salt is crystalline.

3. The omarigliptin acid addition salt according to claim 1 or 2, wherein the omarigliptin acid addition salt is selected from the group of omarigliptin hydrochloride, omarigliptin fumarate, omarigliptin *R*-mandelate, omarigliptin S-mandelate and omarigliptin *RS-*mandelate.

4. The omarigliptin acid addition salt according to claim 3, which is crystalline omarigliptin hydrochloride **characterized by** having a powder X-ray diffractogram comprising reflections at 2-Theta angles of (18.7 ± 0.2)°, (21.0 ± 0.2)° and (24.2 ± 0.2)°, when measured with Cu-Kalpha₁,₂ radiation at a wavelength of 0.15419 nm and a temperature in the range of from 20 to 30 °C.

5. The omarigliptin acid addition salt according to claim 3, which is crystalline omarigliptin fumarate **characterized by** having a powder X-ray diffractogram comprising reflections at 2-Theta angles of (13.8 ± 0.2)°, (19.7 ± 0.2)° and (21.2 ± 0.2)°, when measured with Cu-Kalpha₁,₂ radiation at a wavelength of 0.15419 nm and a temperature in the range of from 20 to 30 °C.

6. The omarigliptin acid addition salt according to claim 3, which is crystalline omarigliptin *R*-mandelate **characterized by** having a powder X-ray diffractogram comprising reflections at 2-Theta angles of (6.3 ± 0.2)°, (17.9 ± 0.2)° and (21.9 ± 0.2)°, when measured with Cu-Kalpha_{1,2} radiation at a wavelength of 0.15419 nm and a temperature in the range of from 20 to 30 °C.

7. The omarigliptin acid addition salt according to claim 3, which is crystalline omarigliptin S-mandelate **characterized by** having a powder X-ray diffractogram comprising reflections at 2-Theta angles of (16.3 ± 0.2)°, (18.6 ± 0.2)° and (20.7 ± 0.2)°, when measured with Cu-Kalpha_{1,2} radiation at a wavelength of 0.15419 nm and a temperature in the range of from 20 to 30 °C.

8. The omarigliptin acid addition salt according to claim 3, which is crystalline omarigliptine *RS*-mandelate **characterized by** having a powder X-ray diffractogram comprising reflections at 2-Theta angles of (6.3 ± 0.2)°, (12.7 ± 0.2)° and (18.6 ± 0.2)°, when measured with Cu-Kalpha_{1,2} radiation at a wavelength of 0.15419 nm and a temperature in the range of from 20 to 30 °C.

9. The omarigliptin acid addition salt according to any one of the preceding claims, **characterized by** a molar ratio of omarigliptin and acid in the range of from 1.0:0.8 to 1.0:1.2.

10. A process for the preparation of an omarigliptin acid addition salt, preferably of a crystalline omarigliptin acid addition salt according to any of claims 1 to 9 comprising:
(i) Providing the free base of omarigliptin in amorphous form, pseudo-amorphous form, crystalline form or as a mixture of two or more of these forms;
(ii) Preparing a mixture comprising the omarigliptin provided in step (i) and a solvent system comprising at least one alcohol;
(iii) Reacting the free base of omarigliptin comprised in the mixture provided in (ii) with an acid to obtain an acid addition salt of omarigliptin
(iv) Separating the omarigliptin acid addition salt, preferably the crystalline omarigliptin acid addition salt of step (iii) from the reaction mixture.

11. The process according to claim 10, wherein in (iii) the acid is selected from the group of hydrochloric acid, fumaric acid, *R*-mandelic acid, S-mandelic acid and *RS*-mandelic acid.

12. Use of the omarigliptin acid addition salt according to any of claims 1 to 9 for the preparation of a pharmaceutical composition.

13. A pharmaceutical composition comprising an effective amount of the omarigliptin acid addition salt of any of claims 1 to 9 and at least one pharmaceutically acceptable excipient.

14. The pharmaceutical composition according to claim 13 for use as a medicament.

15. The pharmaceutical composition according to claim 13 for use in the treatment of a condition selected from diabetes type 2, obesity and high blood pressure.
